# FASCICULE DE BREVET EUROPEEN

(11) **EP 2 314 669 B1**
(45) Date de publication et mention de la délivrance du brevet: **31.12.2014**
(21) Numéro de dépôt: 10366005.6
(22) Date de dépôt: 07.10.2010
(51) Int. Cl.: C12N 1/20, C12R 1/065, C12N 1/22, C05F 11/08

(54) **Substrats matriciels carbones pour l'obtention de bactéries biofertilisantes**
Kohlenstoff-Matrixsubstrate zum Erhalt von Biodünger-Bakterien
Carbon matrix substrates for obtaining biofertilising bacteria

(30) Priorité: 26.10.2009 FR 0905120
(43) Date de publication de la demande: 27.04.2011
(73) Titulaire: Agronutrition, 31390 Carbonne (FR)
(72) Inventeur: Claude, Pierre-Philippe, 54000 Nancy (FR)
(74) Mandataire: Lassiaille, Christian Michel

(56) Documents cités:
- WO-A1-90/02719
- WO-A2-03/046156

## Description

### ÉTAT DE LA TECHNIQUE ET PROBLEMATIQUE

### Endo-génécité et agro-pédo-climats (APC)

L'endogénécité des biomasses microbiennes réintroduites dans un sol est déterminante pour l'efficacité relative de ces biomasses. En effet, et malgré un certain vague entourant la biogéographie microbienne des sols (Curtis et al. 2002 ; Fierer et Jackson 2006 ; Cho et Tiedje 2000), l'importance de *cibler* la réintroduction de biomasses microbiennes biofertilisantes demeure. Ce ciblage contribue à leurs plus grandes efficacités, et/ou à simplifier les aspects réglementaires entourant leurs réintroductions dans leurs milieux d'origines.

Cette *endogénécité* implique une *concordance* entre les milieux (sols) d'origines et ciblés, *i.e*. une certaine *similarité* entre son APC d'origine et de réintroduction. Il sera ainsi possible de situer la parcelle dans un APC ; la souche/biomasse (azoto)bactérienne provenant de cet APC sera donc nécessairement « endogène » lorsque réintroduite par biofertilisation. Par APC nous voulons dire ici un ensemble d'unités cartographiques et/ou de pédons dont les principales caractéristiques physico-chimiques (pH, C-organiques, P, K, texture, etc.) permettent de les regrouper, avantageusement par voie d'analyse multi-variées telle que *l'analyse par composantes principales,* ou ACP, avec d'autre unités cartographiques jugées ainsi suffisamment similaires. A noter que les APC ainsi formés ne sont pas nécessairement contigües ; cette non-contigüité étant ici particulièrement révélatrice de la biogéographie des communautés bactériennes du sol (Fierer et Jackson 2006).

Afin de mieux exploiter en agronomie ce phénomène de concordance APC-spécifique et *d'endogénicité* des biomasses microbiennes réintroduites, il eut été nécessaire - d'une part, de caractériser plus systématiquement la SAU (surface agricole utiles) en APC repérables et - d'autre part, d'inventer un protocole d'obtention de biomasses microbiennes APC-spécifiques plus performant et reproductible ponctuellement pour chaque parcelles agronomique ciblés. En ce sens, j'ai cherché dans le passé à concevoir des cultures mères de bactéries telluriques (FR0115542 ; voir *infra*), et cela afin de reproduire *in vitro* l'essentiel de ces caractéristiques physico-chimiques d'un sol appartenant à un sol donné, voire à terme un APC. Les biomasses ainsi obtenues et réintroduites dans leurs sols d'origines furent effectivement plus performantes que leurs consoeurs obtenues plus conventionnellement et/ou réintroduites dans un autre type de sol, vraisemblablement appartenant à un autre APC. En accord avec ces données, notez que le principal facteur structurant les sols arables est la teneur en argile, le carbone organique (*C_{org}*) et le pH ; il n'est donc pas surprenant que les bactéries habitant dans des complexes argilo-humique dont la formation est fonction de la teneur en C_{org} et le pH du sol soient mieux adaptées à la vie dans leurs sol d'origine qu'autrement.

### Adaptation des bactéries à la vie dans les sols

Il est tentant, d'un point de vue stratégique et méthodologique de vouloir réduire l'adaptation à la vie dans les sols à quelques mécanismes simples, notamment l'adhésion des bactéries aux surfaces solide et abiotique. En effet, et malgré les prétentions de Syn et al. 2004 qui laissent entendre avoir pu ramener le phénomène de l'oligotrophie rustique des *Pseudomonas in situ* à quelques gènes, trois pour être exacte, Tao et al. 1999 révèlent clairement que l'espression de près de 10% des quelques 4000 gênes d'*E.coli* est modulée en réponse à une oligotrophie imposée. Des quelques gênes de Syn et al. 2004, aux quels centaines selon Tao et al. 1999, le débat reste ouvert, pour le moins dire.

La sélection et l'isolement de souches bactériennes du sol les mieux adaptées à la vie dans les sols (AVS) pour la fabrication d'inocula de bactéries favorisant la croissance des plantes (BFCP) est donc problématique puisque l'indentification de génotypes stables de telles BFCP adaptées à la vie dans les sols (« fit in soil » *cf.* Mirleau *et al.* 1999) est difficilement contrôlable. (Par adaptation à la vie dans les sols je veux dire ici la qualité qu'on les souches BFCP candidates à persister activement dans les sols arables, avantageusement en proximité des résidus de culture enfouis et/ou des racines, suffisamment longtemps et en nombre suffisant pur avoir un effet phytogène détectable.) Cette habilité qu'ont les bactéries AVS n'est pas sous le contrôle d'éléments et/ou même de programmes génétiques compréhensibles, voire simples (Klausen *et al* 2006, Sauer 2003). Le profilage génomique (*génotypage*) et/ou métabolique des souches BFCP les plus AVS reste, pour l'essentiel, à accomplir. Pire, cette caractérisation s'avérerait vraisemblablement complexe et pourrait prendre la forme d'un vague profil génomique (*eg*. Rüberg *et al.* 2003), et cela sans pour autant identifier précisément les quelques mécanismes biomoléculaires les plus déterminants. L'AVS demeur, d'un point de vue génomique, un concept abstrait et mal défini (Klausen *et al.* 2006).

Cela dit, ces bactéries les plus adaptées en ce sens mettent en oeuvre des mécanismes favorisant l'adhérence et la formation de micro - colonies, ainsi que la communication (*cf.* signalisation intercellulaire) contribuant à l'organisation « sociale » de ces populations en biofilms (Klausen *et al* 2006 ; Sauer 2003). Faute de programmes génétiques compréhensibles et/ou de « profil d'expression génomiques » reproductibles, ces quelques mécanismes, et les composantes génétiques qui les sous-tendent, peuvent en principe servir de critères de sélection. En effet, les *Azotobacteraceae* les plus adaptées à la vie dans les sols seront, en principe, sauront le plus facilement s'organiser en biofilms du fait d'une meilleure communication intercellulaire via la synthèse et l'excrétion desdits facteurs d'auto-induction. En effet, selon Ganter et al. 2006 les cellules bactériennes impliquées dans la formation de tels biofilms ont en théorie intérêts à percevoir de faibles concentrations des facteurs tels que l'homo-sérine-lactones n-acétylés (*n*HSL). De telles molécules étant impliquées dans l'organisation coloniale des cellules bactériennes dites « pionnières » lors de la formation de biofilms, lesdits biofilms étant une partie intégrante de l'environnement immédiat des complexes argilo-humique de sol.

### Les BFCP les plus prometteuses sont difficilement extractibles du sol du fait qu'elles sont avantageusement copio - et oligotrophes

Les BFCP, les plus adaptées à la vie dans les sols sont difficilement maintenues actives *in vitro* (Barer et Harwood 1999) du fait qu'elles sont oligotrophes, rustiques et donc incapables de dominer numériquement les autres cellules introduites *in vitro.* Cette rusticité des bactéries les plus AVS fait qu'elles n'intègrent pas de façons prévisibles et manipulables les contingents bactériens d'origine tellurique. Du coup, les isolats bactériens les plus dominants en milieux de culture *in vitro* sont souvent peu ou pas apparentés à l'essentiel de la microflore bactérienne du sol (Torsvik *et al.* 1990a et b ; Ward *et al.* 1990).

Cette dominance numérique de souches copiotrophes au dépends de souches génétiquement distinctes qui le sont moins est connue (Dunbar *et al* 1997 ; Bastians *et al.* 2000 ; Ferrari *et al.* 2006 et 2005 ; Zengler *et al.* 2002 ; Janssen *et al.* 2002 ; Simu et Hagström 2004 ; Stevenson *et al* 2004 ; Cho et Giovannoni 2004 ; Tamaki *et al.* 2005 ; Connan et Giovannoni 2002 ; Shimonura *et al.* 2006). Cependant, ce phénomène de domination numérique de souches copiotrophes (« *enrichement bias* » ; Dunbar *et al* 1997) n'a jamais été expressément cité comme responsable de la relative inefficacité des inocula BFCP. De plus, et paradoxalement, ces approches sont difficilement applicables à des bioprocédés. En effet, elles sont trop biaisées vers la sélection de bactéries strictement oligotrophes. Il en résulte la sélection et la mise en culture de cellules normalement incultivables *in vitro,* mais dont les taux de croissance *in vitro* sont trop faibles pour assurer la rentabilité de leurs productions industrielles. Ces méthodes (i.e. Ferrari *et al.* 2006 et 2005 ; Zengler *et al.* 2002 ; Janssen *et al.* 2002 ; Simu et Hagström 2004 ; Stevenson *et al* 2004 ; Cho et Giovannoni 2004 ; Tamaki *et al.* 2005 ; Connan et Giovannoni 2002 ; Shimonura *et al.* 2006) cherchent surtout à démonter que certains spécimens peuvent, malgré leurs rusticité et/ou leurs oligotrophie, être cultivés *in vitro.*

Selon Van Gestel *et al.* (1993) il existe une différence entre les cellules rustiques dites « autochtones » et les cellules plus copiotrophes dites « zymogènes » (sensu Ponchon 1958) en ce qui concerne leurs niveaux d'activités métaboliques et d'AVS. Selon ces auteurs, les bactéries zymogènes moins rustiques proviennent surtout des familles *Pseudomonacea" Azotobacteraceae* et *Rhizobiaceae,* tandis les bactéries autochtones plus rustiques elles sont surtout membre des familles *Arthobacter* (*Micrococcaceae*). Par exemple, Boylen (1973) affirme que la stratégie des bactéries coryniformes pour mieux survivre dans les sols consiste surtout à « hiberner » en réduisant leur taux de métabolisme, tout en faisant apparaître d'épaisses couches (« manteaux ») de peptidoglycans. Les bactéries plus zymogènes, elles, miseraient surtout sur une production plus ponctuelle d'EPS et d'LPS (*i.e*. lipo - polysaccharides), l'adaptation d'un mode de vie plus benthique, et éventuellement l'enkystement de leurs cellules. Par exemple, l'oligotrophie des BFCP rustique *in situ* est associée à la surproduction d'exopolysaccharides (Roberson *et al.* 1993 ; Deflaun *et al*. 1990). En effet, cette surproduction nui à la prolifération *in vitro* desdites BFCP, bien qu'elle leurs profitent *in situ.*

Pourtant, sonder les CAH à l'aide l'oligo-nucléotides afin d'identifier les BFCP les plus adaptées à la vie dans les sols sans recourir à des pré-cultures aqueuses est aujourd'hui physiquement impossible. Sonder une suspension aqueuse de ces particules de sol est toute aussi inefficace puisque l'essentiel des BFCP les plus adaptées à la vie dans les sols sont associées aux fines particules de sols et aux CAH ; elles seront nécessairement exclues de cette suspension, surtout si elle est diluée pour fin d'isolement d'unités formatrices de colonies (UFC) bactériennes. Enfin, l'inoculation de milieux de culture très pauvres en substrats carbonés (Janssen *et al*. 2002), sans pour autant relâcher les liens qui unissent les BFCP les plus AVS aux CAH, ne fera que favoriser la rétention et la mise en culture de souches rustiques, mais du coup excessivement oligotrophes et difficilement productibles industriellement.

### « Diauxie » et compromis oligo/copiotrophes ; une spécificité des Azotobacteraceae

Dans les faits, et selon Lynch (1984 ; Plant and Soil, 76 : 307-318) des microorganismes réintroduits véritables phytogènes seront tant autochtones (oligotrophes) que zymogènes (copiotrophes). Pratiquement, ces souches incarnent un compromis avantageux entre une oligotrophie rustique, caractéristique des espèces *Arthrobacter* du sol par exemple, et une copiotrophie zymongène, caractéristiques de beaucoup de souches bactériennes *Pseudomondaceae,* y compris donc les *Azotobacteraceae.* Ces souches sont donc robustes mais aussi relativement productibles *in vitro ;* elles sont les membres de consortia les plus copiotrophes de contingents oligotrophes.

Les bactéries du sol pouvant réaliser cet avantageux compromis oligo / copiotrophie pourraient effectivement faire appelle à deux (2) types de substrats ; (i) dans un premier temps au cour de la phase oligotrophe un substrat non glucidique plus récalcitrant, et (2) dans un deuxième temps au cours de la phase copiotrophe un substrat glucidique, voire même de simples oses, plus facilement métabolisables. Or, ce type de croissance bi-phasique est caractéristique de microorganismes dont la croissance s'effectue en présence de deux substrats carbonés, et donc l'utilisation de l'un d'eux est préférentiel, l'utilisation du deuxième ne commençant vraiment qu'après l'épuisement du premier. Ce type de croissance bi-phasique est aussi appelée « croissance auxique » (Moreno et Vargas-Garcia 1995).

Ce phénomène est particulièrement applicable aux *Azotobacteraceae* en présence de pHBA et de glucose (Moreno et Vargas-Garcia 1995), l'utilisation du glucose étant ici préféré dans un premier temps à celle du pHBA plus récalcitrant. Or, en sols arables - contrairement au mode opératoire *in vitro* proposé par Moreno et Vargas-Garcia 1995, les teneurs de pHBA et d'autres acides phénoliques sont largement supérieures à celles du glucose, la croissance oligotrophique à base de pHBA devrait donc *in situ* et en principe précéder la croissance copiotrophique du fait d'un enrichissement progressif et très relatif en glucose (oses) de la solution du sol, par exemple suite à l'enfouissement et la dégradation des résidus de culture pailleux. C'est en ce sens que cette diauxie pHBA / glucose est ici vraisemblablement à l'orgine de ce compromis oligo / copiotrophie des *Azotobacteraceae* tant recherché.

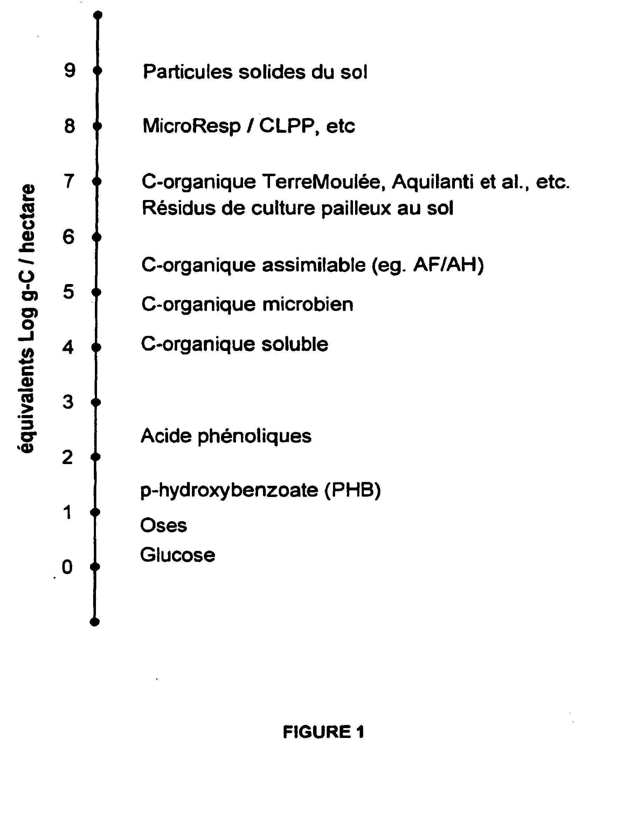

La matière organique du sol étant en partie composée d'acides phénoliques condensées, les teneurs des sols en acides phénoliques est nécessairement de l'ordre de 10⁷ g/ha (en surface sur 10 cm de profondeur). Or, l'équivalent de 1% de celles-ci sont effectivement en solution, soit environ 10⁴ à 10⁵ g /ha (*idem*). La teneur en oses de cette matière organique est elle au moins un ordre de grandeur plus faible que celle des acides phénoliques ; *cf.* Figure 1 pour une représentation schématique des ces proportions. A noter que pour ce qui concerne les oses, seulement 1% de ceux-ci sont effectivement en solution, soit au plus 10 à 20 kg d'oses en solution du sol par hectare (*idem* ; voir les données d'Itoh et al. 2007, à titre d'exemple).

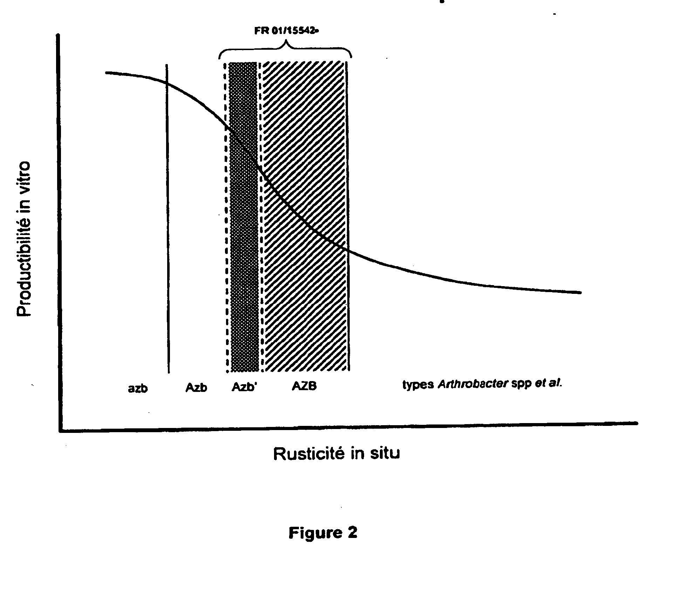

Il existe donc vraisemblablement un certain rapport entre les teneurs en C_{organique} soluble du sol et celle des composés phénoliques, plus particulièrement le susdit pHBA, rapport favorable au développement rapide des *Azotobacteraceae* les plus utiles au sens entendu. Plus précisément, il est convenu que, même faibles, les teneurs en pHBA de la plupart des sols arables est supérieure à celles des simples oses, notamment le glucose (Figure 2). La dilution d'un échantillon de sol abaissera cette teneur en pHBA, mais aussi et dans une même proportion celles des susdits oses, et cela en dessous d'un certain seuil d'environ 74 à 100 uM. Plus précisément - et pour ce qui concerne les *Azotobacteraceae,* les ratios de ces quelques dizaines de kg d'acide phénoliques par rapport au quelques kg d'oses en solution par hectare est d'autant plus conséquent. Plus exactement, c'est la teneur relative d'une des acides phénolique en particulier - l'acide *para*-hydroxy-benzoïque (pHBA), en présence d'oses solubles qui affectera la mise en marche de la diazotrophie azotobactérienne (Moreno et Garcia-Vargas 1995).

### Revue de quelques protocoles d'obtention de bactéries telluriques rustiques

Au risque de généraliser grossièrement, il existe trois type de protocoles de mise en culture de bactéries récalcitrantes à la culture *in vitro* ;
i) par dilution successive d'un quelconque milieu de culture enrichi de façon à établir des conditions de mise en culture franchement oligotrophiques
ii) par diffusion d'une « calque » de la composition nutritionnelle et oligoélémentaire vers un support de culture inerte, généralement une membrane microporeuse, avantageusement en polycarbonate
iii) par formation *in vitro* d'un biofilm, généralement d'origine aquatique (marin, sédiments, eaux potables, etc.)

Les travaux de Janssen *et al*. (2002) ainsi qu'un récent brevet (WO 2003/042351) mentionnent la mise en culture *in vitro* de bactéries dites à croissance lente (« *slow growing* ») et cela malgré la présence et la forte concurrence des bactéries dites à croissance rapide (« *fast growing* »), ou « copiotrophes ». Ce brevet concerne surtout la mise en culture de bactéries oligotrophes provenant d'échantillons d'eau. Or, les souches obtenues avec ces approches (Janssen et al. 2003 ; WO 2003/042351) sont bio-industriellement peu ou pas conséquents puisque les cellules bactériennes ainsi obtenues sont si peu copiotrophes qu'elles ne peuvent être produites économiquement

Bastiaens *et al.* (2002) utilisent une surface solide immergée dans un milieu de culture riche conventionnel. Cependant, et contrairement à FR0115542, ladite surface solide est une membrane hydrophobique imprégnée d'hydrocarbures ; la membrane n'est pas conçue comme l'analogue de l'environnement édaphique, et encore moins comme celui de son CAH.

Il existe aussi quelques méthodes, à base de membranes carbonées, à la différence des cultures mère à base de gel siliciques selon FR0115542, permettant la mise en contacte d'une solution saline extraite du sol et donc calquée sur celui-ci, et de bactéries supposément oligotrophes (Ferrai et *al.* 2005, 2006). Bien que ces méthodes, dites « SSMS » (*soil slurry membrance systems*), permettent de mettre en culture des isolats distincts, variés et précédemment dits « incultivables » *in vitro,* rien ne laisse croire que ces isolats sont particulièrement AVS et productibles au sens entendu ci-dessus. De plus, les dites SSMS ressemblent étrangement au procédé décrit dans le brevet *al*lemand DE 2158827 décrivant une méthode pour la conservation et l'utilisation de bactéries qui consiste à mettre en contacte un milieu de culture adapté et une membrane microporeuses.

Aagot *et al.* (2001) mentionnèrent l'importance de ne pas enrichir excessivement les milieux de culture *in vitro* devant servir à l'isolement de bactéries AVS. Or, Aagot *et al.* (2001) ne cherchent qu'à établir le seuil de dilution d'une des composantes d'un milieu de culture conventionnel, *i.e*. l'acide casaminique, au-delà du quel les cellules plutôt oligotrophes peuvent croître au dépends des cellules copiotrophes. La caractérisation des divers isolats ainsi obtenus à l'aide d'oligosondes, notamment via la technique dite de REP-PCR, ne sert qu'à démontrer que les souches isolées en milieux de culture dilués sont différentes de celles isolées en milieux de culture non - dilués, sans pour autant affirmer qu'elles sont du coup plus rustiques et adaptées à la vie dans les sols.

Accessoirement, les travaux d'Aagot *et al.* (2001) démontrèrent l'inutilité de la technique d'isolement, dite parfois de la « terre moulée » (*cf*. Pochon 1954), pour l'obtention de souches BFCP véritablement rustiques et bien adaptées à la vie dans les sols. En effet, l'enrichissement outre mesure du milieu d'origine, d'un sol reconstitué avec du glucose par exemple, afin de faire proliférer une composante de la flore bactérienne, notamment les *Azotobacteraceae,* ne favorise pas l'obtention de souches BFCP résistantes à divers stresses, osmotique, alcaline, *etc.,* caractéristique de la vie dans les sols. Favoriser le repérage de souches *édaphiquement* compétentes à partir d'échantillons de sols *via* la méthode de la « terre moulée » est donc inefficace parce qu'elle favorise la prolifération de cellules bactériennes plutôt copiotrophes (zymogènes), opportunistes à l'égard de ce type de substrats carbonés apportés massivement.

La perturbation desdits « microhabitats » (Grundmann et al. 2001) abolira la compétition qui pèse normalement sur ces différentes populations bactériennes. Cette abolition permettra à des bactéries normalement très discrètes *in situ* de croître librement *in vitro* au dépends des bactéries plus représentatives et mieux adaptées à la vie dans les sols. Grundmann et al. 2001 proposent donc une « micro-fragmentation » et un « micro - échantillonnage » du sol. Cette micro - fragmentation des micro-habitats intactes évite de mettre en concurrence des microorganismes qui, à l'échelle microscopique du moins, n'interagiraient peu ou pas directement ; cette ségrégation spatiale peut favoriser la croissance *in vitro* de microorganismes autrement peu ou pas enclins à le faire étant donné la susdite compétition. Cela dit, cette méthode est difficilement transposable à la bioindustrie du fait de sa technicité capricieuse.

L'utilisation d'oligosondes destinées à la détection spécifique de genre et d'espèce de BFCP n'est pas en soi nouvelle. En effet, selon la technique décrite dans EP 1130115 il est aujourd'hui possible de puiser directement à même la diversité bactériologique du sol et d'y retrouver spécifiquement des *Azospirillum,* et *Herbaspirillum, etc.* Cependant, cette sélection, faute d'avoir aujourd'hui en main une caractérisation suffisamment poussée des mécanismes qui sous-tendent l'adaptation à la vie dans les sols ne peut pas en soi garantir (assurer) que les souches ainsi détectées seront suffisamment rustiques pour constituer des inocula BFCP efficaces, d'où la nécessité de produire les susdites pré-cultures.

Il y a aussi la technique dite CARD-FISH (Ferrari *et al.* 2005, 2006) principalement destinée à l'isolement de *bactérioplanctons* oligotrophes, mais aussi en principe applicable aux bactéries telluriques. Celle-ci, telle qu'appliquée par Ferrari *et al.* (2005, 2006) consiste à repérer sur des SSMS (*soil slurry membrane systems*), homologues en sorte des CAH et des CMBT tel que proposé dans FR0115542, les cellules rustiques et oligotrophes et tenter de progressivement mettre en culture un nombre de celles-ci. Cependant, et encore une fois, la formation des SSMS imprégnées d'extrais de sol arables implique la destruction partiale du CAH afin de mettre en suspension les cellules bactériennes, procédé de destruction biaisé à l'avantage des cellules bactériennes probablement les moins intimement associées aux CAH.

Enfin, il existe des mises en culture (en milieux riches et donc normalement réservés aux cellules copiotrophes) de bactéries et bactérioplanctons marins (Simu et Hagström 2004 ; Cho et Giovannoni 2004). Il existe aussi des brevets concernant des « supports de récupération » (WO 99/02649), des « méthodes de sélection » (EP 0106504 A2) ou encore des « procédés favorisant la croissance des microorganismes des sols » (WO 99/09834) toutes aussi inadaptées à la production des susdits inocula BFCP. Généralement, le but de ces protocoles est d'augmenter le nombre, la diversité, voire la rapidité d'isolement de souches bactériennes telluriques pouvant être cultivées *in vitro ;* ce n'est qu'accessoirement que ces cellules pourraient être parfois adaptées à la vie dans les sols.

### FR0115542 : une avancée technique majeure, mais imparfaite

FR0115542 concerne l'obtention de BFCP adaptées à la vie dans les sols via la formation d'un biofilm analogue de la zone hydratée adjointe aux complexes argilo-humiques des sols. Ce biofilm est une sorte de « culture mère de bactéries telluriques » (CMBT). Les BFCP issues de ces populations, avantageusement *Azotobacteraceae, Pseudomanceae* et/ou *Rhizobiaceae,* sont plus efficaces (*i.e*. plus phytogènes) par rapport à l'ensemble des souches témoins. FR0115542 représente en ce sens une avancée notable puisqu'elle permet de sélectionner parmi les cellules bactériennes telluriques retenues à la surface des CMBT des cellules BFCP vraisemblablement rustiques mais aussi suffisamment copiotrophes pour croître en milieux de culture *in vitro* enrichis.

Cependant, étant donné que les CMBT n'abritent vraisemblablement pas que des souches bactériennes adaptées à la vie dans les sols ce processus de sélection définitive par comparaison de leurs activités phytogènes à celles des susdites souches témoins, bien qu'efficace en soi, reste néanmoins fastidieux. En effet, les quelques souches non - adaptées plutôt copiotrophes, présentes initialement dans les aliquotes provenant des CMBT peuvent déplacer les souches plus adaptées lors de cultures liquides en milieux enrichis Afin de minimiser ce déplacement, j'ai prévu une manipulation décrite dans FR0115542 ; cette manipulation est elle aussi imparfaite.

En effet, FR0115542 préconise la réitération de nombreuses pré-cultures en milieux culture électifs par voie de restaurations de ces milieux avec des aliquotes provenant des CMBT, et cela au risque de voir apparaître à tout moment des cellules bactériennes plus copiotrophes pouvant contaminer la préparation. Pire, l'AVS étant à ce jour mal expliquée, FR0115542 ne peut servir directement au contrôle de la qualité des inocula BFCP ainsi produits ; l'efficacité relative de ces inocula BFCP est donc confirmée qu'en fin de parcours à l'aide d'essais phytogène par rapport à une gamme de souches témoins en principe moins AVS. Il semble que l'oligotrophie caractéristique des BFCP candidates présentes dans la phase aqueuse surnageant les CMBT ne soit pas toujours conservée lors de ces réitérations. Ce faisant, l'avantageux compromis oligo- / copio-trophie des souches candidates, définition même de l'adaptation à la vie dans les sols au sens de FR0115542, ce défait, et des souches BFCP moins adaptées en ce sens risquent d'être retenues. Cela dit, les inocula BFCP prototypes préparés à l'aide de FR0115542 se sont avérés efficaces *in situ* (Claude et Fillion 2004, Claude et Giroux 2006).

### DIVULGATION DE L'INVENTION

### Problème technique

D'une part le problème technique provient du fait que les *Azotobacteraceae* sont - bien que quasi universellement présentes, très minoritaires en sols arables - de l'ordre d'une *Azotobacteraceae* pour 1 million ses congénères non-*Azotobacteraceae.* D'autre part, les cellules (azoto)bactériennes du sol les mieux adaptées à la vie dans les sols arables recevant régulièrement des apports agronomiques de résidus de culture pailleux sont aussi métaboliquement très réactives dès l'apparition de quelques dizaines - 50 à 100, umoles de glucose par litre de solution du sol. Or, bien qu'il est en principe relativement facile de favoriser la dominance microbiologique d'une certaine, ou de certaines populations azotobactériennes, *in vitro* en éliminant *a priori* toutes formes de N immédiatement assimilable, ce micro- (nano- ?) dosage d'oses est plus problématique du fait qu'il est difficile de déterminer précisément la teneur en ose de la solution du sol à un moment précis, la « durée de vie » (temps de résidence) du glucose dans les sols étant très courte, soit de l'ordre de quelques dizaines minutes au plus. En ce sens, l'apport de *micro-doses* à une solution nutritive sans N inoculée à partir d'un aliquote de sol ne permettra pas d'assurer le non - dépassement de ces seuils - somme tout très faibles (de l'ordre de 50 à 100 uM), permettant aux souches *Azotobacteraceae* les plus *compétentes* au sens entendu de démarrer métaboliquement les premières.

Or, bien que l'essentiel des bactéries les mieux adaptées à la vie dans les sols sont associées aux fines particules de sols et aux CAH, il est difficile de les séparer, mécaniquement et/ou chimiquement. De plus, le haut niveau d'adaptation à la vie dans les sols attribuable à la rusticité des BFCP candidates est métaboliquement trop complexe pour servir de base à un profilage génomique définitif de ces BFCP oligotrophes que nous devons extraire des complexes argilo - humiques, voire avantageusement des CMBT.

Enfin, l'état de la technique ne procure aucune assurance en ce qui a trait à la rusticité des cellules bactériennes d'origine tellurique et/ou leurs efficacités éventuelles entant que BFCP. Pour faire simple, disons que les méthodes d'obtention de souches telluriques rustiques proposées à ce jour visent surtout le caractère oligotrophe des BFCP les plus rustiques ; ce faisant, les cellules bactériennes ainsi isolées sont trop souvent excessivement oligotrophes et donc difficilement productibles économiquement par bioprocédés conventionnels, et donc de mauvaises candidates pour servir de base à la fabrication industriel d'inocula BFCP, fabrication qui suppose malgré tout une certaine prolifération *in vitro* de ces isolats.

Bien que FR0115542 représente une avancée technique majeure puisqu'elle permet de retenir en suspension des BFCP candidates pourtant étroitement associées à un analogue *in vitro* du CAH du sol du quel elles sont originaires, cette mise en culture nécessite néanmoins la production de pré - cultures réitératives sources potentielles de contaminations. De plus, FR0115542 ne permet pas de micro-doser l'apparition progressive du glucose - ou d'oses plus généralement, le substrat matriciel sur le quel ce développe le biofilm étant à base de silice ; à terme le milieu surnageant ce gel silicique revient de plus en plus oligotrophe entrainant la dominance microbiologique de souche qui le sont aussi, à l'excès à terme. Les souches (azoto)bactériennes les plus réactives à de (très) faibles (*u*M) concentration d'oses - pourtant en principe très recherchées (voir *supra)* risquent de ne pas pouvoir dominer le moment venu un milieu de culture riche sensé en favoriser la multiplication ; ces quelques souches les plus recherchées seront ainsi déplacées (dominées) par un ensemble de souches soit purement copiotrophes (des contaminants), soit par une population plus importantes de souche (trop) oligotrophes trop rustiques pour afficher cet avantageux compromis entre copio- / oligotrophie caractéristiques des souches les plus adaptées la vie dans les sol au sens entendu ici et donc aussi les meilleures candidates pour la constitution d'inocula biofertilisants.

La fabrication d'inocula BFCP destinés à l'agriculture, et plus particulièrement à la biofertilisation des grandes cultures à l'aide de microorganismes sont donc aujourd'hui sérieusement compromise. Les alternatives qu'auraient pu être, i) l'identification directe des BFCP les plus adaptées à la vie dans les sols sans avoir à s'appuyer *a priori* surtout sur leur caractère oligotrophe au dépends d'une certaine copiotrophie utiles à leur production bioindustrielle, et/ou ii) l'utilisation d'une profilage génomique et/ou métabolique bien défini de leurs rusticité, sont aujourd'hui impraticables étant l'apparente complexité du phénomène qu'est l'adaptation à la vie dans les sols tel définit ci-dessus.

Schématiquement, le problème technique est le suivant (Figure 2) ; comment éviter simplement la rétention des bactéries soit excessivement copiotrophes (azb, Azb) soit excessivement oligotrophes (eg. *Arthrobacter*), et retenir que les souches - dites « AZB » qui incarnent le mieux un compromis avantageux entre ces deux extrêmes ? Il s'agit d'épurer, en sorte, un éventuel contingent de souches composé majoritairement d'AZB obtenues selon FR0115542 mais contenant immanquablement les quelques souches contaminantes moins avantageuses - Azb par exemple. Or, ces contaminants Azb pourraient, du fait de plus grandes copiotrophie, dominer une éventuelle culture d'enrichissement.

### Solution technique proposée

La solution technique a comme prémisse le constat que les teneurs en acide phénoliques - y compris donc celles de pHBA, de la solution du sol sont nécessairement plus faibles que celles des oses / osides. Du coup, la dilution au 5^{ième} ou au 10^{ième} d'un échantillon de sol sera immanquablement et essentiellement dépourvu d'ose - ou du moins d'une molarité suffisamment faible et en dessous des susdits 50 à 100 uM équivalents glucose. En de telles situations, les *Azotobacteraceae* pourront très avantageusement croître en mode diazotrophique étant donné la présence d'un minimum de pHBA ; une fois un minimum de glucose en solution - à hauteur des susdits quelques 50 à 100 *u*M, elles seront majoritairement les premières à pouvoir se manifester comme particulièrement réceptives à d'aussi faibles teneurs en oses - *i.e*. particulièrement adaptées à la vie dans les sols au sens entendu.

Deuxièmement, la présence de para-hydroxy benzoate (pHBA ; ne pas confondre avec le poly-hydroxy-butyrate, ou PHB) est nécessaire au fonctionnement *in situ* de Nif chez les *Azotobacteraceae.* Plus généralement, il est connu que la composition en acides phénoliques de la solution du sol affecte la composition et la taille des populations bactériennes de la résidusphère et du sol (eg. Blum et al. 2000). Cet effet des acides phénoliques, y compris donc de pHBA, peut apparaître en présence de concentrations ouvent bien en dessous de 0,1 umol par g de sol, soit de l'ordre d'une dizaine (10) kg par hectare en surface sur 10 cm. Étant donné la spécificité du rôle de pHBA vis-à-vis les *Azotobacteraceae in situ* (Moreno et Garcia-Vargas 1995) et les quantités concernées - ici donc de l'ordre de quelques kg par hectare, la manipulation de ces concentrations est envisageable d'un point vue agronomique.

Troisièmement, le phénomène dit de « *déclenchage métabolique* » (*metabolic triggering* ; De Nobili *et al.* 2001) est distincte de celui dit de « d'*amorçage métabolique* » (*metabolic priming* ; Kuzyakov *et al.* 2002) et permet à certaines bactéries du sol de réagir fortement (rapidement) à teneur en oses de l'ordre des centaines de ug par kg de sol. Plus précisément, Nguyen et Guckert 2001 ont établi que le Km pour l'utilisation du glucose par les microorganismes du sol - voir Coody et al. 1986 pour une revue de ce concept a *priori* enzymologique mais depuis appliqué à la bactériologie, y compris et plus particulièrement les (azoto)bactéries, est d'environ **74 uM,** soit en termes pondéreux l'équivalent de quelques kg - deux pour être exacte, de glucose par hectare de sol en surface sur 10 cm, soit une bonne ordre de grandeur plus faible que le Km pour l'utilisation du glucose par des racines de maïs. A comparer ici ces quelques kg de glucose aux quelques dizaines kg de pHBA (voir *supra*).

L'invention consiste à combiner ces trois concepts afin d'assurer d'autant plus cette avantageux compromis entre autochtonie (oligotrophie) et zymogénie (copiotrophie) des biomasses *Azotobacteraceae* biofertilisantes recherchées. En effet, la supposée dichotomie autochtone / zymogène - d'une part, et d'autre part cette forte réactivité des bactéries en état de veille métabolique pouvant être déclanchées (*triggered*) en présence de quelques centaines de ug d'oses par kg de sol ont déjà été valorisé ; voir en ce sens FR0115542 et FR0900426. Nous proposons aujourd'hui d'intégrer ces deux inventions à une troisième - FR0600014, afin de développer *de facto* un processus de fermentation à l'état solide (Fes) simple pouvant assurer un avantageux compromis entre la sélection de biomasses *Azotobacteraceae* autochtones et copiotrophes dès la première apparition d'oses issus de la dégradation des résidus de culture pailleux, soit par définition un inocula *Azotobacteraceae* biofertilisant des plus efficace.

Concrètement, l'invention consiste en un **procédé d'obtention** *in vitro* de biomasses bactériennes édaphiques comprenant les étapes de ; (i) mise en contacte d'un échantillon de sol avec un substrat matriciel de manière à former une zone à la surface du substrat analogue à celle adjointe au complexe argilo-humique (CAH) du sol abritant l'essentiel de la composante bactérienne autochtone (du sol), afin de retenir à la surface ou dans le substrat matriciel l'essentiel de la microflore bactérienne endogène aux milieux édaphiques (APC) d'origine, (ii) maturation de cette zone en conditions oligotrophiques permettant aux cellules les plus constitutives du CAH qui ont coloniser ce substrat matriciel de migrer éventuellement vers la phase liquide le surnageant, (iii) récupération et culture des souches bactériennes les plus prolifiques en milieux de cultures liquides riches, à caractères autochtone (oligotrophes), rustiques et persistantes *in situ,* le ***substrat matriciel étant ici carboné et semi-solide*.**

Encore une fois, par APC ici je veux dire ici un groupe homogène d'unités cartographiques représentatives d'au moins une parcelle agronomique chacune suffisamment similaires en termes de leurs principales caractéristiques pédologiques dont la genèse est susceptibles d'avoir été influence par l'évolution du climat environnant. Ces groupes homogènes peuvent être établit à l'aide d'analyses par composantes principale, - entre autres, et/ou divers types d'analyses multi - dimensionnelles. Il existe une certaine base de connaissance à ce sujet.

Afin d'obtenir ce substrat matriciel carboné semi-solide il est loisible d'imprégner d'eau déminéralisée et stérile une matrice cellulosique - un filtre dit « Durieux™ » non-bactéricide par exemple, et de le placer au fond d'une boîte de Petri stérile. Initialement, la quantité d'eau et de matrice sont avantageusement les mêmes, ce qui permet donc de constituer une matrice semi-solide contenant initialement 50% de matière sèche (solide) et 50% d'eau (liquide). A mesure que progressera la période d'incubation ce substrat deviendra de plus en plus solide que liquide et pourra être éventuellement ré-imprégner afin de prolonger cette période d'incubation, incubation dans les faits assimilable ici à une sorte de fermentation à l'état solide.

Le substrat matriciel carboné semi-solide est *a priori* dépourvu d'oses et d'osides libres assimilables par les (azoto)bactéries du sol ; il en libère progressivement lors de sa dégradation. Au cour de cette fermentation à l'état solide, la microflore d'origine tellurique apportée via l'échantillon de sol assurera la plus part du temps la dégradation enzymatique et/ou fongique de la matrice cellulosique. Cette dégradation libera nécessairement - et surtout progressivement, des oses et/ou osides nécessaire au déclencheage métabolique des *Azotobacteraceae* les mieux adaptées à la vie dans les sols au sens entendu (voir *supra).*

L'échantillon de sol est préparé de façon à retenir que les fines particules de sols aux quelles adhèrent les bactéries les mieux adaptées à la vie dans les sols. Par adaptation à la vie dans les sols nous voulons dire ici qu'il y a une certaine concordance (similarité) entre les milieux d'origine et de réintroduction des biomasses (azoto)bactériennes - d'une part, et d'autre part que ces biomasses établissent un avantageux compromis entre leurs caractères oligotrophiques et copiotrophiques, *i.e.* qu'elles sont très réactives à de très faibles teneurs en substrats carbonés assimilables. Cette définition est semblable à celle déjà énoncée dans FR0115542, toute en intégrant ici cette notion de réactivité propre à la théorie dites des « trigger molecules » (De Nobili et al. 2001).

La granulométrie de ces fines particules de sols est d'environ 500 um, plus particulièrement d'au plus 270 um, et avantageusement d'environ 70 à 170 um. Ces fines particules de sols peuvent être obtenues à partir d'un sol - avantageusement comme de raison appartenant à l'APC d'origine, séchés passivement à l'aire libre par tamisage. Étant donnée que la majorité des bactéries du sols son intimement associées à particules plutôt fines du sol, ce fractionnement par tamisage à le double avantage de favoriser l'inoculation du substrat semi-solide, et du coup d'en faciliter le saupoudrage.

Il est important de s'assurer que les proportions substrat matriciel carboné semi-solide : échantillon de sol soient comprises entre 25 et 1, plus particulièrement entre 10 et 3, et avantageusement d'environ 5. Cette proportion avantageuse suppose que les teneurs de la solution du sol en ose - en glucose notamment, n'excède pas 10 kg par hectare, soit pour 10⁹ de sol sur 10 cm de profondeur en surface ; si la teneur de la solution du sol en ose et en ce sens supérieure, la proportion substrat semi - solide : sol est majorée conséquemment à hauteur de 25 : 1.

Enfin, le substrat matriciel carbonés semi-solide ainsi mis en contacte avec lesdits échantillons de sol est incubé suffisamment longtemps pour permettre le développement *in situ* de populations *Azotobacteraceae.* Tel que déjà mentionné, les substrats matriciels carbonés sont périodiquement ré-humidifiés par aspersion de façon à maintenir les susdites proportions à peu près constante en dépit d'une nécessaire évaporation.

Les biomasses *Azotobacteraceae* ainsi obtenues peuvent maintenant être réintroduites dans leur APC d'origine par pulvérisation liquide des résidus de culture au sol. Cette réintroduction peut avantageusement ce faire selon Claude et Fillion 2004, ou encore dans le cas de mélanges ou d'engrais organo-minéraux selon Claude et Giroux 2006.

Concrètement, il s'agit donc de permettre la dilution d'un échantillon de sol dans une substrat matriciel carboné temporairement inerte de façon à assurer la diminution des teneurs (concentrations) en glucoses (oses) en dessous de seuils assurant ainsi que les pHBA deviennent de facto le seuil et/ou principal substrat carboné pour les *Azotobacteraceae.* Pendant la période d'inertie du susdit substrat carboné, les *Azotobacteraceae,* et seulement les *Azotobacteraceae* pourront croître en mode diazotrophe au dépends de ce pHBA ; une fois l'inertie des substrats avantageusement cellulosiques rompue, ces populations azotobactériennes maintenant plus dominantes au sein de ces résidusphères pourront véritablement s'imposées, voire être facilement isolables entant que-t-elles, de ces résdusphères suffisamment peuplées par de telles *Azotobacteraceae* ; à terme, ces résidusphères peuvent elles même faire office de préparation inoculantes. En ce sens, la présente invention permet de renverser l'habituelle diauxie (voir *supra)* imposée aux *Azotobacteraceae* lors de leur obtention par voie d'isolement et de rétention *in vitro,* obligeant celles-ci à utiliser préférentiellement des oses (sucrose ou glucose) dans un premier temps, et des acides phénoliques - notamment pHBA, dans un deuxième temps. La présente invention permet aussi de tenir compte d'un certain Km bactériologique pour le glucose, sans pour autant imposer de facto aux *Azotobacteraceae* indigènes des sols un milieu de croissance aqueux très différent de leur habitat *in situ* au contacte des CAH et/ou des résidusphères, matrices foncièrement solides, elles.

### Avantages apportés

L'invention comporte de nombreux avantages par rapport à l'état de la technique. Par exemple, il n'est plus nécessaire de faire des dilutions extrêmes des substrats carbonés selon Janssen *et a*/. (2003) au risque d'obtenir des cellules dont la principale caractéristique serait d'être de strictes oligotrophes au dépends d'une certaine copiotrophie pourtant utile, voire nécessaire, à leurs valorisations agronomique et bioindustrielle. L'invention permet aussi de mieux assurer le maintient du caractère véritablement édaphique des BFCP candidates retenues *in vitro.* Les souches BFCP ainsi obtenues sont du coup particulièrement bien adaptées à la fermentation à l'état solide, avantageusement selon le protocole décrit dans notre précédente demande de brevet FR0600014, ou encore selon EP 0 406 103 A1. Enfin, du fait qu'elle ne nécessite pas la fabrication d'un gel silicique, et/ou - surtout la restauration réitérative d'une phase aqueuse à sa surface, la mise en oeuvre de l'invention est beaucoup plus simple que celle décrite dans FR0115542.

### BREVE DESCRIPTION DES DESSINS ET FIGURES

- **Figure 1 :**: Disposition sur une échelle logarithmique des équivalents g-C glucose par hectare des diverses fractions organo-minérales du sol, et - à titre de repères, des apports de substrats proposés par les méthodes de « profilage métabolique » des populations microbiennes du sol (*i.e.* MicroResp™, CLPP, etc.) ainsi que des quantités de C glucose qu'implique le Km des bactéries du sol pour le glucose (*i.e*. 74 uM selon Nguyen et Gurkert 2001). A noter que, en réduisant par « dissolution solide-solide » au sens de la présente invention les concentrations de pHBA, les concentrations de C glucose sont elles aussi réduites et ici portées en dessous des susdits 74 uM.
- **Figure 2 :**: Représentation schématique du compromis avantageux entre productibilité *in vitro* et rusticité *in situ* de bactéries du sol, et plus particulièrement des BFCP candidates retenues sur cultures mères de bactéries telluriques (CMBT) selon FR0115542.

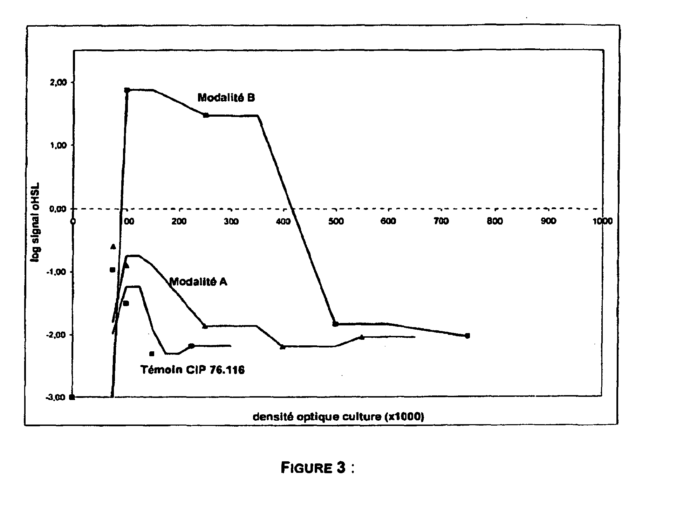

- **Figure 3 :**: Dynamique de production de molécules d'auto-induction, notamment les HSL, en fonction de la densité cellulaire (optique) d'un milieu de culture enrichi et inoculé avec des *Azotobacteraceae* obtenues selon les Modalités A et B au sens de la présente invention, et - en guise de témoin, *A. chroococcum* CIP 76.116. Le signal chromatographique spécifique aux homosérine lactones octanoyl (oHSL) est rapporté en fonction de la densité optique de la culture (azoto)bactérienne.

### MEILLEURE MODE DE REALISATION DE L'INVENTION

A ce stade et afin d'illustrer le meilleur mode de réalisation de l'invention, deux échantillons de sols alsaciens - dits « se13 » et « gewn » (voir Tableau 1), sont été utilisés. Les populations bactériennes obtenues via la présente invention de ces deux sols seront considérées endogènes si elles sont réintroduites dans leurs sols d'origines. À terme, le concept d'APC su sens entendu ici permettra d'étendre cette réintroduction de populations bactériennes endogènes non seulement au sol d'origine en question, mais aussi aux sols jugés suffisamment similaires d'un point de vue multi-variés, par exemple via une analyses par composantes principales d'un ensemble de données agro-pédologiques.

Selon le type de sol et sa teneur en pHBA, un aliquote plus ou moins dilué sera ajouté à une simple FeS de façon à assurer (approcher) un rapport pHBA / oses d'environs 3 à 4, et cela tout en assurant que la teneur en oses de cette FeS soit initialement inférieure à 70 - 100 uM. Initialement - le temps pour les résidus de culture pailleux et/ou tout autre substrat carboné équivalent d'être dégradés et métabolisés par une certaine flore fongique, ce rapport favorisera les *Azotobacteraceae* les plus susceptibles d'être induites par ce *déclenchage métabolique* (DM) à base d'oses et d'acides phénoliques particulières. Les conditions particulières suivantes permettent de réconcilier au sien d'une même FeS ;
- un milieu de culture suffisamment pauvre en oses pour assurer le DM que des souches les plus réactives en ce sens, et donc les plus adaptées à la vie dans le sols arables transitoirement fournis en quantités agronomiques de résidus pailleux ;
- un milieu de culture suffisamment riche en pHBA pour assurer la croissance en absence d'azote apporté des *Azotobacteraceae* les plus autochtones.

Il y a donc aussi une dynamique temporelle partie intégrante de la présente invention ; la mise en oeuvre de cette dynamique est assurée par la présente invention ;
- première période : relative abondance du pHBA par rapport aux oses favorisant la prolifération des *Azotobacteraceae* autochtones ;
- deuxième période : à partir du moment où il y a une certaine dominance des *Azotobacteraceae,* les oses ainsi libérés par la dégradation fongique des résidus de culture pailleux favoriseront la production en masse de telles biomasses ;
- troisième période : croissance quasi exponentielle des populations *Azotobacteraceae* par fermentation à l'état solide, avantageusement modulaire et enrichie de Mo.

En ce sens, à très faibles teneurs en oses - soit de l'ordre de quelques mg par kg de mélanges FeS au sens de FR06/00014, les *Azotobacteraceae* vont plutôt stocker les glucides au lieu de les consommer pour fin de structuration (*i.*e. 1^{ière} période). A mesure que les résidus de culture pailleux sont dégradées, les teneurs en oses vont augmenter progressivement, et cela beaucup plus rapidement que celles des acides phénoliques y compris celle de PHBA (*i.e.* 2^{ième} période). Cela dit, la prépondérance du pHBA par rapport aux autres acides phénoliques dont les concentrations relatives et absolues régressent (voir Martens 2002) favorisera la dominance de plus en plus marquée des *Azotobacteraceae.* A la fin de cette 3^{ième} période - où le rapport pHBA / oses est maintenant d'environs 0,25, ladite FeS sera principalement constituée de souches AZB dont les stockes de glucides sont importants, assurant à celles-ci un bon niveau de robustesse une fois réintroduites dans leurs APC d'origine.

### Production des inocula, avantageusement par fermentation à l'état solide

L'échantillon de sol est séché passivement à température ambiante de façon à maintenir en vie l'essentielle de la flore *Azotobacteraceaea.* La fraction plutôt fine de cet échantillon, soit d'au plus environ 500 um et avantageusement entre 70 et 170 um, est obtenue par tamisage. Cette fraction de fines particules de sol sera intégrée à un substrat cellulosique afin de constituer une fermentation à l'état solide au sens de la présente invention. Ce substrat cellulosique peut être en principe de simples résidus de culture pailleux séchés passivement à température ambiante et hachés et tamisés à 2 mm. Cela dit, ces pailles peuvent aussi abriter certains pathogènes tels que et par exemple *G. graminis var. triticii -* agents pathogène de la maladie des céréales communément appelée piétin verse. Afin d'éviter ce risque phytosanitaire, nous proposons qu'à la fin du susdits processus de fermentation à l'état solide ce pré-mélange contenant une abondance d'*Azotobacteraceae* soit traité avec une dose convenable de fongicide capable d'éliminer toutes les propagules vivantes de telles agents pathogènes.

Or, la teneur en oses de tels résidus pailleux est *a priori* inconnue, et pourrait rompre cette équilibre pHBA / oses au sens de la présente invention. Je propose donc d'utiliser de la cellulose pure tout aussi attaquable par la susdite flore fongique, mais - initialement du moins, dépourvue d'oses ; à ce mélange 1/5 de fines particules de sol et de cellulose pure, nous pouvons ajouter un extrait aqueux des résidus de culture pailleux-disons au 25^{ième} de façon a éviter l'apport d'oses,. Cela suffira à inoculer cette cellulose avec des propagules fongiques cellulolytiques qu'abrite nécessairement de tels résidus, et aussi avec les quelques *Azotobacteraceae* qu'elles pourraient aussi contenir. Un inoculum fongique cellulolytique peut aussi être utilisé, notamment si celui-ci est obtenu de tels résidus de culture pailleux.

Concrètement et par exemple, 2,5 g de cellulose pure peuvent être mélangés à 0,5 g de fines particules de sol. On ajoute ensuite 2,5 ml d'eau - avantageusement déminéralisée mais contenant en plus du susdit extrait aqueux une solution nutritive diluée adaptée à une telle fermentation à l'état solide (voir *infra*). A noter que le cellulose étant à ce stade insoluble, les fines particules de sol sont effectivement « diluées » au 5^{ième} en solution - ou plutôt en suspension, aqueuse. Au fur et à mesure que cette fermentation progresse, cette eau sera consommée et/ou évaporée, tandis que la cellulose elle sera dégradée en métabolites plus soluble ; du coup, les fines particules de sol demeureront toujours en présence de 5 fois plus de ce volume réactionnel, soit en accord avec une des prémisse de la présente invention.

La susdite solution nutritive pourra avantageusement contenir un minimum de Mo. La présence de ce minimum de Mo favorisera la sur - accumulation de Mo par les *Azotobacteraceae* de façon à ce que celles-ci deviennent des vecteurs de ce Mo, tant vers les populations *Azotobacteraceae in situ* que vers - éventuellement, la culture en place.

Enfin, ce procédé de fermentation à l'état solide ainsi que les inocula qui en résultent, peuvent avantageusement être conditionnés « modulairement » selon les principes de nos précédentes inventions rapportées dans FR0505753 et FR0600014. Cette modularité favorisera tant la production, la manutention, la conservation et l'utilisation par le client de ces préparations.

### Méthodes d'analyses et dispositifs expérimentaux

### Dispositifs expérimentaux et analyses statistiques

Lors d'essais phytogènes, j'ai disposé *unités expérimentales* en carrés latins 4 x 4. L'essai comporte des préparation inoculantes issues de ces fermentations à l'état solide, chaque unité expérimentale sera composée d'une de ces quatre modalités intégrée à l'un et l'autre des deux types de sol - gewn et se13, gewn étant ici le sol d'origine des fines particules de sols ; en principe donc les *Azotobacteraceae* ainsi réintroduites seront endogènes à gewn mais non à se13. Une telle disposition des unités expérimentales appariant soit les modalités de fermentation à l'état solide sans et avec (-/+) Mo lors, soit les inocula issues de ces modalités (avec Mo) sur ou hors du sol d'origine *(i.e.* gewn), permet d'apprécier les différences entre Modalités A, B, C et leur témoin non-inoculé, soit en terme absolu (*i.e.* %N des fermentations à l'état solide et/ou MSPA et MOB-élémentaire de *Lolium multiflorum*), soit en terme relatif (*i.e*. le rapport entre les deux éléments appariés de chacune des unités expérimentales). En ce sens, il s'agit ici d'un dispositif avec parcelles imbriquées au sein d'une même unité expérimentale, cette dernière étant répétée quatre fois et disposée en carré latin.

### Dosage et caractérisation microbiologique des Fes APC-spécifiques

La dominance et le peuplement azotobactérien des Fes-APC spécifiques peuvent être facilement établis à l'aide de milieux de culture sélectifs pour *Azotobacteraceae* (eg. Kumar et Narula 1998), Il est aussi possible de déterminer - par rapport à un substrat matriciel cellulosique avec lesdites fines particules de sol (seule source d'azote *a priori* non incubé), la teneur en Nₜₒₜₐₗ de cette fermentation à l'état solide à termes (*i.e*. ici de 3 à 4 semaines à titre expérimental, mais dans les faits facilement ramenée à quelque jours en situation bioindustrielle plus réaliste et optimisée ; eg. Zhang et al. 2004) - toute augmentation en Nₜₒₜₐₗ étant ici nécessairement attribuable à une forme ou une autre de diazotrophie azotobactérienne.

### Évaluation de l'effet phytogène :

Les biomasses azotobactériennes ont été apportées au sol via la bactérisation de résidus de cultures à hauteurs de 1 % p/p enduits de l'équivalent d'environ 1^{e}13 cellules / ha, soit - 1 million de cellules par g de résidus de culture, dose-hectare est comparable à celle de Claude et Fillion (2004) et Claude et Giroux (2006). Dans un premier temps, l'accumulation, en mg par pot de 2 L, sur une période de 30 jours de la matière sèche des parties aériennes de plantules d'avoine (*Avena sativa* L.) a servit à établir l'activité phytogène des différents consortia de souches BFCP. Nous avons aussi évalué la repousse de plantules de ray-grass (*Lolium multiflorum* Lam.) à 10 jours d'intervalle sur une période de 64 jours, la première coupe (C₁) intervenant à environs 28 jours post-semis. Les *matières sèches par les parties aériennes* (MSPA) ainsi produites sont, une fois l'activité photosynthétique résolue, remobilisées, « translocalisées » vers les divers organes de stockages dont le poids est généralement déterminant pour les rendements. Les matières fraîches sont ainsi récoltées et étuvées à 65 degré C pour 48 heures afin de déterminer leur teneur en humidité. Enfin, le dosage des teneurs en N, P, K, Ca, Mg et oligoéléments permet par la suite de déterminer la mobilisation végétale de ces éléments.

### Indices du degré d'adaptation à la vie dans les sols :

*i) **essai phytogène endo- et exo-géniques*** : Afin de démontrer que les biomasses *Azotobacteraceae* réintroduites dans leurs APC d'origine - ici le sol dit gewn (Tableau 1), sont ainsi plus efficaces (phytogènes) entant que biofertilisants que de telles biomasses « exogènes » introduites dans un APC différent de leur APC d'origine, - ici le sol dit se13 (Tableau 1), j'ai installé en serre un dispositif expérimental en ce sens ; voir *infra* un exemple d'application de la présente invention.
ii) ***dosage de la production de facteurs d'auto-induction** :* Les teneurs en facteurs d'auto-induction - notamment les homosérine lactones *n*-acétylés (*n*HSL), responsables en partie de la rusticité et de la formation de biofilms par les cellules les mieux adaptées à la vie dans les sols, peuvent être déterminées selon Huang et al. (2003), Leadbetter et Greenberg (2000), voire Flagan et al. (2003) et rapportées graphiquement par rapport à la densité cellulaire, évaluée grossièrement selon sa densité optique à 600 nm. Il est aussi possible d'utiliser une méthode avec injecteur APS™ permettant l'introduction directe dans le chromatographe d'échantillons solides (eg. les matrices cellulosiques), liquides (eg. les surnageants des milieux de culture liquide des biomasses azotobactériennes ainsi obtenues), voire pâteux (eg. les culots de centrifugation des ces milieux de culture contenant donc que lesdites biomasses azotobactériennes). Les échantillons sont analysés par chromatographie en phase gazeuse couplée à la spectrométrie de masse en mode impact électronique.

### APPLICATION INDUSTRIELLE ET AGRONOMIQUE

Afin de démontrer que la présente invention est susceptible d'être appliquée en agronomie, nous avons mené trois (3) expérimentations ;
(i) une concernant la production par fermentation à l'état solide d'une série de préparations inoculantes à base de substrats cellulosiques afin de démontrer la plus grande activité des *Azotobacteraceae,* et donc du coup de l'accumulation de l'azote d'origine diazotrophiques dans ces préparations ;
(ii) l'autre afin de démontrer la plus grande efficacité relative des inocula ainsi produits s'ils sont réintroduits dans leurs APC d'origine d'où proviennent les fines particules de sols ayant servies à leurs fabrications.
(iii) une troisième démontrant cette fois-ci une certaine (sur)production de facteurs d'auto-induction - plus particulièrement ici d'homosérines lactones (HSL) et plus précisément encore d'octanoyl-HSL (oHSL), par les biomasses azotobactériennes obtenues selon la présente invention par rapport à une souche référence de l'institut Pasteur (*A*. *chroococcum* CIP 76.116) obtenue plus conventionnellement.

***(i) Fabrication des préparations inoculantes** :* A des substrats cellulosiques nous avons ajouté l'équivalent de 20% p/p de fines particules de l'un des deux sols alsaciens (Tableau 1) dit « gewn ».

**Tableau 1 : Caractérisation physico-chimique des sols (APC) utilisés.**

| Paramètre | Unité | « APC » se13 | « APC » gewn |
|---|---|---|---|
| Argile | % | 21,1 | 27,0 |
| LF/LG | Na | 1,10 | 1,72 |
| Sables fins | % | 4,8 | 18,1 |
| pH (eau) | Na | 6,6 | 5.7 |
| Matière organique | g/kg-sol | 21,5 | 55,5 |
| CEC | mÉq/kg-sol | 128 | 101 |
| Saturation CEC | % | 100 | 56 |
| P₂O₅ assimilable | mg/kg-sol | 200 | 37 |
| K₂O échangeable | mg/kg-sol | 110 | 80 |
| MgO échangeable | mg/kg-sol | 140 | 200 |
| CaO échangeable | mg/kg-sol | 3140 | 1850 |
| Zn-DTPA | ug/kg-sol | 1400 | 700 |

J'ai ajouté 0,5 g de ces fines particules de sol - tamisées à sec à 270 um, à 2,5 g d'une matrice cellulosique, ici de simples filtres de cellulose (« Durieux »). J'ai simplement saupoudrer à la surface de ces filtre hydratés à l'aide de quelques 2,5 ml d'eau déminéralisée - il est aussi possible d'utiliser ici une solution nutritive faiblement dosée dépourvue d'azote, et contenant une fois sur deux (voir *infra)* une dose de Mo équivalente à 1 g de Mo par hectare (*i.e*. un *nano*grame (ng) de Mo par g de sol), apportée ici *via* les susdits 2,5 mL (g) de milieu aqueux dosée à 3,5 mM de Mo.

Afin de favoriser le *déclenchage métabolique -* et cela au sens de de Nobili et al. (2001) par exemple, des *Azotobacteraceae* les plus adaptées à la vie dans les sols en proximité des résidusphères, nous avons dosé en glucose la susdite solution nutritive à hauteur de 74 uM (cf. Nguyen et Guckert 2001 ; op cit FR 09/00428) pour la modalité B, 0 uM pour la modalité A, et 740 uM pour la modalité C. Nous avons donc trois (3) modalités - A, B et C, avec et sans la susdite *nano*-dose de Mo sensée favoriser le développement et la dominance des *Azotobacteraceae* en de telles situations où le rapport C/N des substrats cellulosiques sont très élevés ;
Modalités A : cellulose avec 0 uM glucose et +/- Mo
Modalités B : cellulose avec 74 uM glucose +/- Mo
Modalités C : substrat cellulosique avec 740 uM glucose +/- Mo

Ces mélanges à base substrats cellulosiques, de fines particules de sols, de glucose et de Mo contiennent maintenant 3 g de solides (cellulose ou résidus pailleux et fines particules de sol) et 3 ml (g) d'une phase aqueuse plus ou moins enrichie de glucose et/ou de Mo. Une fois la fermentation à l'état solide menée à terme, plus de 75-80% de l'eau aura été consommée et/ou évaporée, ainsi qu'une partie des substrats cellulosiques minéralisée et réorganisée en biomasses *Azotobacteraceae* ; le poids final de la préparation inoculante ainsi préparée sera donc de l'ordre d'environ 3 g, soit ici plus qu'amplement de quoi inoculer - pour fin d'expérimentation à ce stade, les 3,5 g de résidus de culture pailleux intégrés à 350 g de deux types de sols différents (Tableau 1) secs reconstitués.

Cela dit, les susdits 12 à 15 g de préparation inoculante seront re-suspendus dans 150 mL d'eau déminéralisée (dilution 10⁻¹), tandis que seulement 1 mL de cette suspension d'un litre (apport de 10⁻³) sera utilisé pour inoculer les susdit 3,5 g de résidus de culture pailleux (*i.e*. 1 / 3,5) ; le facteur de dilution de la préparation inoculante par rapport aux résidus de culture pailleux ciblés est donc de 3,5 x 10⁻⁴. Or, il faut envisager de traiter 10⁷ g de résidus de culture pailleux au sol par hectare *in situ* ; la préparation inoculante éventuelle produite bio-industriellement devra donc permettre une augmentation du titre par g des biomasses *Azotobacteraceae* par un facteur de 100 par rapport à une certaine préparation inoculante prototype telle que préparé ici. Cette augmentation est parfaitement envisageable techniquement étant donné l'état de la technique (eg. Zhang et al. 2004).

Les populations *Azotobacteraceae* par g de substrat cellulosique selon les différentes modalités (A, B ou C) sont à peu près comparables - soit de l'ordre de 10⁹ cellules par g, et nettement plus grandes en présence de Mo. A titre indicatif de l'activité de cette nitrogenase azotobactérienne au sein des différentes matrices cellulosiques, j'ai aussi dosé l'azote total (Nₜₒₜₐₗ) de ces préparations inoculantes avant leurs dilutions pour fin d'ensemencement des résidus de culture pailleux (voir infra). Encore une fois, il n'y a pas de grandes différences à ce stade entre les différentes modalités appliquées à chacun des sols ; elles affichent toute environ 0,10% d'Nₜₒₜₐₗ par g de préparation inoculant (*i.e*. de matrice cellulosique incubée avant dilution), et environ deux fois plus en présence de Mo. Un éventuel effet phytogène des ces différentes modalités (A, B ou C) avec Mo (voir *infra*) ne pourra pas être attribué à une différence de titre cellulaire et/ou d'azote réduite apporté au début de l'essai au moment de l'inoculation.

***(ii) essai phytogéne des préparations inoculantes ;*** Le Mo au sein des fermentations à l'état solide favorise le développement de la flore azotobactérienne. C'est ici avec la modalité B (matrice cellulosique + 74 uM glucose et micro-dose Mo) que la flore azotobactérienne semble être la plus phytogène, voire la plus adaptée à la vie dans les sols en proximité des résidusphère, *i.e*. capable d'une certaine oligotrophie toute en étant très réactive à des apparitions transitoires de très faibles molarité d'oses. De plus - fait notable, cette adaptation à la vie dans les sols est surtout appréciable si le sol ciblé est similaire - voire identique, au sol d'origine d'où proviennent les susdites fines particules de sol partie intégrante de la susdite fermentation à l'état solide. En effet, pour vérifier cette double hypothèse - (i) la supériorité de la modalité B par rapport aux modalités A et C, et (ii) sa plus grande efficacité relative sur sol gewn d'où proviennent les susdites fines particules de sol par rapport à un autre type de sol, par exemple ici se13 (Tableau 1), nous avons installé l'essai en serre suivant.

Les modalités A, B et C avec Mo et appliquées au sol gewn, ainsi qu'un témoin sans inoculation, ont été re-suspendues - les quatre répétitions de l'essai (i) étant ici combinées dans 4 L d'eau déminéralisée (*i.e*. 15 g de préparation inoculante pour 150 mL d'eau), soit une dilution de 10⁻¹ ; 1 mL de cette suspension servant la suite à inoculer 3,5 g de résidus de culture pailleux (blé). En supposant une dose-hectare d'un kg d'inoculum, ce taux d'inoculation pratiqué ici équivaut à l'inoculation *in situ* de 3,5 x 10⁵ g de résidus de culture pailleux au sol par hectare, soit entre 15 et 25 fois moins qu'en situations agronomiques où les retours de pailles et/ou de chaumes sont de l'ordre de 5 à 8 x 10⁶ g par hectare. Or, cette disproportion sera en pratique et selon l'état de la technique dans le secteur des fermentations à l'état solide facilement corrigée. En effet, les titres cellulaires de ce type de fermentation à l'état solide peut facilement atteindre 10¹⁰ cellules *Azotobacteraceae* par gramme (eg. Zhang et al. 2004).

Les susdits 3,5 g de résidus de culture pailleux ainsi inoculés avec chacune desdites modalités A, B et C (avec Mo) et la préparation témoin non-inoculante sont intégrés par malaxage à 350 g de sol sec et reconstitué par tamisage à 4 mm. Deux (2) type de sols de deux APC différentes - « gewn » (APC no. 29) et « se13 » (APC no. 40) (Tableau 1), le sol gewn étant ici le sol d'origine d'où proviennent les susdites fines particules de sol intégrées aux fermentations à l'état solide selon les modalités A, B et C. Une fois intégrés au sol, les mélanges sol / résidus de culture pailleux inoculés sont placés dans des pots de 0,5 L ; 4 répétitions (Figure 3) pour chacune des modalités essayées sur sol gewn et se13 sont installées. Une quarantaine de graines de *Lolium multiflorum -* soit pondéralement 0,20 g, sont répandues à la surface de chaque pot, et recouvertes d'une certaine quantité de sable siliceux inerte. Les pots sont irrigués avec une solution nutritive contenant cette fois-ci un minimum d'azote minéral (12 mg-N_{NO3NH4} par kg de sol) de façon à éviter aux plantules des carences en N, mais sans pour autant nuire au démarrage de la diazotrophie des *Azotobacteraceae*. Cette solution contient aussi un complément de Mo destiné à la plante, l'alimentation en Mo des *Azotobacteraceae* étant elle assurée par le Mo apporté pendant la fermentation à l'état solide.

Les MSPA du *Lolium* sont récoltées 28 et 56 jours post-semis, séchées à 55-60 degrés C, pesées et analysées pour leurs teneurs en N, P, K et Zn afin d'en déterminer la mobilisation par plantule. Le dosage élémentaire est effectué par le laboratoire d'analyse de la Sadef (www.sadef.fr) selon les normes Afnor adoptées à le Cofrac. Tel qu'attendu, et comme nous pouvons le voir au Tableau 2, les MSPA et leurs mobilisation d'N, P, Mg et Zn sont supérieures - par rapport au témoin, en présence de biomasses *Azotobacteraceae* obtenues via les Modalités A, B ou C - notamment *via* la Modalité B, et cela d'autant plus si elles sont réintroduites dans leurs APC d'origine (gewn) qu'autrement dans un autre APC (se13).

**Tableau 2 : Production de matière sèche des parties aériennes (MSPA) par plant de Lolium multiflorum et sa mobilisation de N, P, Mg et Zn 25 et 56 jours post-semis selon le type de azotobactérisation des résidus de culture pailleux, i.e. soit endo- (sur « gewn ») soit exogène (sur « se13 ») et la modalité de préparation des biomasses Azotobacteraceae.**

| | mg-MSPA / plant | | ug-N / plant | | ug-P / plant | | ug-Mg / plant | | ug-Zn / plant | |
|---|---|---|---|---|---|---|---|---|---|---|
| | gewn | se13 | gewn | se13 | gewn | se13 | gewn | se13 | gewn | se13 |
| 28 jours post-semis | | | | | | | | | | |
| témoin sans inoculation | 32 | 31 | 264 | 266 | 88 | 84 | 40 | 69 | 3,33 | 2,98 |
| inoculation via Fes Modalité A | 41 | 32 | 265 | 267 | 91 | 89 | 40 | 37 | 3,21 | 3,04 |
| inoculation via Fes Modalité B | 47 | 40 | 325 | 298 | 102 | 96 | 48 | 38 | 3,89 | 3,24 |
| inoculation via Fes Modalité C | 38 | 33 | 274 | 371 | 95 | 94 | 42 | 34 | 3,24 | 3,17 |
| 56 jours post-semis | | | | | | | | | | |
| témoin sans inoculation | 11,6 | 10,6 | 97,5 | 93,4 | 31,5 | 28,3 | 14,5 | 23,8 | 1,26 | 1,09 |
| inoculation via Fes Modalité A | 13,7 | 11,1 | 100,1 | 93,6 | 33,0 | 32,2 | 13,6 | 14,0 | 1,13 | 1,11 |
| inoculation via Fes Modalité B | 15,9 | 14,2 | 117,4 | 99,4 | 34,2 | 32,6 | 18,0 | 13,6 | 1,32 | 1,12 |
| inoculation via Fes Modalité C | 13,4 | 11,2 | 95,6 | 140,6 | 35,1 | 34,9 | 15,8 | 11,2 | 1,11 | 1,19 |

***(ii) (sur)production de facteurs d'auto induction (nHSL) ;*** En raison d'une plus grande efficacité relative des inocula préparés selon la Modalité B - *i.e*. incubés initialement en présence 74 uM-glucose, sur sol gewn représentatif de l'APC d'origine de ces biomasses *Azotobacteraceae* que sur sol se13 représentatif d'un autre APC, j'ai voulu démontrer plus directement la plus grande adaptation à la vie dans les sols de ces biomasses par rapport à des biomasses azotobactériennes obtenues plus conventionnellement. Pour ce faire, j'ai comparé la production d'nHSL - et plus exactement d'n-octanoyl HSL par les biomasses produites selon les modalités A et B à celle d'une souche de référence de la collection de l'institut Pasteur (CIP) ; 71.116 (*A. chroococcum*).

La présence de facteurs d'auto-induction n'est détectée que dans les surnageants des milieux de culture liquides d'enrichissement ; les matrices cellulosiques et/ou les biomasses en elles mêmes en sont dépourvues. Cela démontre clairement que ces facteurs d'auto-induction sont produits par les cellules après leurs multiplication par fermentation à l'état solide ; elles pourront vraisemblablement l'être aussi *in situ* une fois ces biomasses réintroduites dans leur APC d'origine. En principe, nous nous attendions que les souches supposément les mieux adaptées à la vie dans les sols produisent le plus de tels facteurs d'auto-induction en début de culture, notamment ici des N-*octanoyl*-homosérine-lactones (Figure 5).

Nous avons simplement dosé (voir *supra*) les oHSL d'un milieu de culture d'enrichissement en *Azotobacteraceae* à t = 1, 2, 3, 4 et 6 heures. La recherche spécifique de l'ion m/z 143 permet de mettre en évidence la présence de oHSL dans tous les surnageants provenant des milieux de culture CIP 76.116, Modalité A et Modalité B ; la concentration la plus élevée est observée dans le surnageant de la Modalité B ; de plus une trace de N-hexanoyl homoserine lactone (hHSL) est également détectée. La mesure des aires de l'ion m/z 143 permet d'estimer la proportion de oHSL dans chaque surnageant en fonction du temps. Les aires des signaux sont rassemblées dans le Tableau 3 ;

**Tableau 3 : Signal m/z 143 caractéristique de l'octanoyl homosérine lactone (oHSL) en fonction du temps d'incubation des diverses biomasses azotobactérienne en milieux d'enrichissement (dépourvu a priori d'azote minéral).**

| temps (t = ) | CIP 76,116 | Modalité A | Modalité B |
|---|---|---|---|
| 1 | 0,1079 | 0,1266 | 29,1125 |
| 2 | 0,0311 | 0,0136 | 0,0145 |
| 3 | 0,0049 | 0,0064 | 0,0000 |
| 4 | 0,0065 | 0,0091 | 0,0093 |
| 6 | 0,0000 | 0,0082 | 0,0000 |
| Moyenne | 0,0301 | 0,0328 | 5,8273 |
| log (moyenne) | -1,5217 | -1,4844 | 0,7655 |

Selon le mode opératoire décrit, ces cellules bactériennes obtenues au sens de la présente invention seraient plus adaptées à la vie dans les sols que les cellules issues de 76.116, et devraient donc (sur)produire plus d'nHSL ; voire en ce sens *supra* Ganter et al. 2006. Or, c'est précisément ce que j'ai pu observer (Figure 3). En effet, pour de faibles densités optiques - indices plus ou moins appropriés de la densité cellulaire, les cellules bactériennes obtenues selon la présente invention produisent nettement plus de nHSL que les bactéries de références 76.116. Les cellules issues de la modalité B (*i.e*. incubés initialement en présence de 74 uM-glucose) produisent encore plus d'nHSL que celles issues de la modalité A (*i.e*. incubés initialement en présence de 0 uM-glucose). Cette surproduction d'nHSL à faible densité de populations azotobactériennes ne peu que réduire d'autant plus la *calling distance* (Ganter et al. 2006) et augmenter ainsi le degré d'adaptation à la vie dans les sols arables de ces cellules azotobactériennes.

### Références bibliographiques

Aagot, Nina Ole Nybroe, Preben Nielsen, et Kaare Johnsen. (2001). An Altered Pseudomonas Diversity Is Recovered from Soil by Using Nutrient-Poor Pseudomonas-Selective Soil Extract Media. Appl. Environ. Microbiol. 67 : 5233-5239.
Bastiaens, L, D. springael, P. Wattiau, H. Harms, R Dewachter, H. Verachtert et L. Diels. 2000. Isolation of Adherent Polycyclic Aromatic Hydrocarbon (PAH)-Degrading Bacteria Using PAH-Sorbing Carriers. Appl. Environ. Microbiol. 66 : 1834-1843.
Barer, M.R. et C.R. Harwood. 1999. Bacterial viability and culturability. Adv. Microbiol. Physiol. 41:94-137.
Blum, Udo, Karen L. Staman, Laura J. Flint. 2000 et Steven R. Shafer. 2000. Induction and/or selection of phenolic acid-utilizing bulk-soil and rhizosphere bacteria and their influence on phenolic acid phytotoxicity. J. Chem. Ecol. vol. 26, no. 9
Boylen, C.W. 1973. Survival of Arthrobacter crystallopoeites during prolonged periods of extreme desiccation. J. Bacteriol. 113(1): 33-37.
Cho, Jae-Chang et James M. Tiedje. 2000. Biogeography and degree of endemicity of fluorescent pseudomonas strains in soil. Appl. Environ. Microbiol. p. 5448-5456 vol. 66, no. 12
Cho, J-C. et S.J. Giovannoni. 2004. Cultivation and growth characteristics of a diverse group of oligtrophic marine Gammaproteobacteria. Appl. Environ. Microbiol. 70 :432-440.
Claude, P-P. (1997). Valorisation des Résidus de Cultures et Biofertilisation : importance et rôle de la persistance active des souches bactériennes réintroduites. Laboratoire d'ingénierie agronomique, INPT - École nationale supérieure d'agronomie de Toulouse (Ensat), (novembre 1997).
Claude, P-P. et L. Fillion. 2004. Effet de l'apport d'un inoculum bactérien aux résidus de culture de maïs-grain au sol sur le rendement et la qualité de blés d'hiver panifiables en France. Agrosol 15(1) : 23-29.
Claude, P-P et M. Giroux. 2006. Effet des engrais organo-minéraux inoculés (EOMI) sur la croissance des plants de maïs-grain, les rendements, les prélèvements des éléments nutritifs et la qualité des grains. Agrosolutions Novembre 2006 No. 17(1) : 51-64.
Connon, S.A. Et S.J. Giovannoni. 2002. High throughput methods for culturing microorganisms in very low nutrient media yield diverse new marine isolates. Appl. Environ. Microbiol. 68:3878-3885.
Coody, Peter N., Lee E. Sommers et Darrell W. Nelson. 1986. Kinetics of glucose uptake by soil microorganisms. Soil Biol. Biochem. Vol. 18, no. 3, pp. 283-289
Curtis, Thomas P., William T. Sloan, et Jack W. Scannell. 2002. Estimating prokaryotic diversity and its limits. Pp. 10494-10499 PNAS vol. 99 no. 16
De Flaun, M.F., A.S. Tanzer, A.L. McAtter, B. Marshall et S.B. Levy. 1990. Development of an adhesion assay and characterization of a adhesion-deficient mutant of Pseudomonas fluorescens. Appl. Environ. Microbiology. 56(1): 112-119.
De Nobili, M., M. Contin, C. Mondini, P.C. Brookes. 2001. Soil microbial biomass is triggered into activity by trace amounts of substrate. Soil Biol. Biochem. 33 : 1163-1170
Dunbar, j., s. white et L. Fornay. 1997. Genetic diversity through the looking glass : effect of enrichment bias. Appl. Environ. Microbiol. 63(4)f:1326-1331.
Ferrari, B.C., S.J. Binnerup et M. Gillings. 2005. Microcolony cultivation on a soil substrate membrane system selects for previously uncultured soil bacteria. Appl. Environ. Microbiol. 71:8714-8720.
Ferrari, B.C., N. Tuluja, K. Stoner et S. Kjellerberg. 2006. Catalyzed reporter deposition -fluorescence in situ hybridization allows for enrichment-independent détection of microcolony-forming soil bacteria. Appl. Environ. Microbiol. 72 :918-922.
Fierer, N. et R.B. Jackson. 2006. The diversity and biogeography of soil bacterial communities. Pp. 626-631 PNAS 17, vol. 103, no. 3
Flagan, S., W-K. Ching et J.R. Leadbetter. 2003. Arthrobacter strain VAI-A utilizes acyl-homoserine lactone inactivation products and stimulate quorum signal biodegradation by Variovorax paradoxus. Appl. Environ. Microbiol. 69:909-916.
Gantner, S., M. Schmid, C. Durr, R. Schuhegger, A. Steidle, P. Hutzler, C. Langebartels, L. Eberl, A. Hartmann et F.B. Dazzo. 2006. In situ quantitation of the spatial scale of calling distances and population density-independentn-acylhomoserine lactone-mediated communication by rhizobacteria colonized on plant roots. Fems microbiol ecol 56 : 188-194
Grundmann, G. L. A. Dechesne, F. Bartoli, J. P. Flandrois, J. L. Chasse et R. Kizungu. 2001. Spatial modeling of nitrifier microhabitats in soil. Soil sci. Soc. Am. J. 65:1709-1716
Huang, J.J., A. Petersen, M. Whiteley et J.R. Leadbetter. 2006. Identification of QuiP, the product of gene PA1032, as the second acyl-homoserine lacton acylase of Pseudomonas aeruginosa PA01. Appl. Environ. Microbiol. 72 :1190-1197.
Itoh, K., A. Watanabe, K. Tsutsuki et S. Kuwatsuka. 2007. Monosaccharide composition of four humus fractions in an andosol and a cambisol. Soil Sci. Plant. Nutr. 53 : 7-11
Janssen, P.H., Penelope S. Yates, Bronwyn E. Grinton, Paul M. Taylor, et Michelle Sait. (2002). Improved Culturability of Soil Bacteria and Isolation in Pure Culture of Novel Members of the Divisions Acidobacteria, Actinobacteria, Proteobacteria, and Verrucomicrobia. Appl. Environ. Microbiol 68 : 2391-2396*.*
Klausen, M., M. Gjermansen, J-U. Kreft et T.Tolker-Nielsen. 2006. Dynamics of development and dispersal in sessile microbial communities: examples from Pseudomonas aeruginosa and Pseudomonas putida model biofilms. FEMS Microbiol. Lett. 261: 1-11.
Kumar, Vivek et Neeru Narula. 1999. Solubilization of inorganic phosphates and growth emergence of wheat as affected by Azotobacter chroococcum mutants. Biol Fertil Soils (1999) 28 :301-305
Kuzyakov, Y. 2002. Factors affecting rhizosphere primint effects. J. Plant. Nutr. Soil Sci. 165 : 382-396.
Leadbetter, J.R. and E.P. Greenberg. 2000. Metabolism of acyl-homoserine lactone quorum-sensing signals by Varivorax paradoxus. J. Bacteriol. 182: 6921-6926.
Martens, Dean A. 2002. Relationship between plant phenolic acids released during soil mineralization and aggregate stabilization. Soil sci. Soc. Am. J. 66:1857-1867.
Mirleau, P., S. Delorme, L. Philippot, J.M. Meyer, S. Mazurier et Ph. Lemanceau. 2000. fitness in soil and rhizosphere of Pseudomonas fluorescens C7R12 compared with a mutant affected in pyoverdine synthesis and uptake. FEMS Microbiol. Ecol. 34 : 35-44.
Moreno, J. et C. Vargas-Garcia. 1995. Growth and nitrogenase activity of Azotobacter vinelanidii in chemically defined media containing glucose and p-hydroxybenzoic acid. Chemosphere 31, no. 2, pp. 2605-2610
Nguyen, C. et A. Guckert. 2001. Short-term utilisation of 14c-[u]glucose by soil microorganisms in relation to carbon availability. Soil Biol. Biochem. 33 : 53-60
Pochon, J. 1954. Manuel technique d'analyses microbiologique du sol. MAsson et Cie., eds., Paris.
Pochon, J. et Y-T. Tchan. 1948. Précis de microbiologie du sol : principes, technique, place dans les cycles géo-biologiques. Masson et Cie, eds., Paris.
Roberson, E.B., C. Chenu et M.K. Firestone. 1993. Microstructural changes in bacterial exopolysaccharides during desiccation. Soil Biol. Biochem. 25(9): 1299-1301.
Rüberg, S., Zhe-Xian Tian., E. Krol, B. Linke, F. Meyer, Y. Wang, A. Pühler, S. Weidner, A. Becker. (2003). Construction and validation of a Sinorhizobium meliloti whole genome DNA microarray: genome-wide profiling of osmoadaptive gene expression. J. Biotechnol. 106 : 255 - 268.
Sauer, K. 2003. The genomics and proteomics of biofilm formation. Genome Biology 2003, 4:219
Shimomura, Y., R. Ohno, R. Kawai et K. Kimbara. 2006. Method for assessment of viability and morphological changes of bacteria in the early stage of colon formation on a simulated natural environment. Appl. Environ. Microbiol. 72:5037-5042.
Simu, K. et A. Hagström. 2004. Oligotrophic bacterioplankton with a novel single-cell life strategy. Appl. Environ. Microbiol. 70:2445-2451.
Stevenson, B.S., S.A. Eichorst, J.T. Wertz, T.M. Schmidt, et J.A. Breznak (2004). New Strategies for Cultivation and Detection of Previously Uncultured Microbes Appl. Environ. Microbiol. 70 : 4748-4755.
Syn, Chris K. C., Jon K. Magnuson, Mark T. Kingsley, et Sanjay Swarup. 2004. Characterization of pseudomonas putida genes responsive to nutrient limitation. Microbiology, 150, 1661-1669
Tao, H., C. Bausch, C. Richmond, F.R.. Blattner et T. Conway. 1999. Functional genomics: expression analysis of escherichia coli growing on minimal and rich media. J. Bacteriol. 181 : 6425-6440
Tamaki, H., Y. Sekiguchi, S. Hanada, L. Nakamura, N. Nomura, M. Matsumura et Y. Kamagata. 2005. Comparative analysis of bacterial diversity in freshwater sediment of a shallow eutrophic lake by molecular an improved cultivation-based techniques. Appl. Environ. Microbiol. 71:2162-2169.
Torsvik, V.; J. Goksoyr et F.D. Daae. 1990a. High diversity in DNA of soil bacteria. Appl. Environ. Microbiol. 56:782-87.
Torsvik, V.; K. Salte; R. Sorhein et J. Goksoyr. 1990b. Comparison of phenotypic diversity and DNA heterogeneity in a population soil bacteria. Appl. Environ. Microbiol. 56:776-781.
Van Gestel, M., R. Merckx et K. Vlassak. 1993. Microbail biomass responses to soil drying and rewetting: the fate of fast- and slow-growing microorganisms in soils from different climates. Soil Biol. Biochem. 25(1): 109-123.
Ward, D.M.; R. Weller et M.M. Bateson. 1990. 16S rRNA sequences reveal numerous uncultured microorganisms in a natural community. Nature 345:63-65.
Zengler, K., G. Toledo, M. Rappé, J. Elkins, E.J. Mathur, J.M. Short et M. Keller. 2002. Cultivating the uncultured. PNAS 99 :15681-15686.
Zhang, X., H. Zhao, J. Zhang et Z. Li. 2004. Growth of Azotobacter vinelandii in a solid-state fermentation of technical lignin. Bioresource. Technol. 95(2004) 31-33

### Liste des abréviations

- APC: agro-pédo-climat
- ACP: analyse par composantes principales
- Afnor: Association française de normalisation
- ALS: argiles, limons, sables
- AVS: adaptation à la vie dans les sols
- AZB: Azotobacteraceae
- BDAT: Base de données des analyses de terre
- BFCP: bactéries favorisant la croissance des plantes
- CAH: complexe argilo-humique
- CARD-FISH: Catalyzed Reporter Deposition-Fluorescence In Situ Hybridization
- CEC: capacité d'échange cationique
- CIP: Collection de l'Institut Pasteur
- CMBT: culture mères de bactéries telluriques
- Cofrac: Comité français d'accréditation
- DM: *déclenchage métabolique*
- EPS: exo-polysaccharide
- FeS: fermentation à l'état solide
- GISSOL: Groupement d'intérêt scientifique sur les sols
- HSL: homo-sérine lactones
- LPS: lipo-polysaccharides
- MO: matière organique
- MOB-: mobilisation (élémentaire)
- MSPA: matières sèches des parties aériennes
- Nif: nitrogenase
- pHBA: para-hydroxy benzoate (para-hydroxy benzoic acid)
- PHB: poly-hydroxy butyrate
- REP-PCR: repetitive element PCR (polymerase chain reaction) fingerprinting
- Sadef: Société alsacienne pour le développement de la fertilisation
- SAU: superficie agricole utile
- SSMS: soil slurry membrane system
- UFC: unité formatrice de colonie (microbiologie)

## Revendications

1. Procédé d'obtention *in vitro* de biomasses bactériennes édaphiques comprenant les étapes de ; (i) mise en contacte d'un échantillon de sol avec un substrat matriciel de manière à former une zone à la surface du substrat analogue à celle adjointe au complexe argilo-humique (CAH) afin de retenir à la surface ou dans le substrat matriciel l'essentiel de la microflore bactérienne endogène aux milieux édaphiques d'origine, (ii) maturation de cette zone en conditions oligotrophiques permettant aux cellules les plus constitutives du CAH qui ont colonisées ce substrat matriciel de migrer éventuellement vers la phase liquide le surnageant, (iii) récupération et culture des souches bactériennes les plus prolifiques en milieux de cultures liquides riches, à caractères autochtone, rustiques et persistantes *in situ*, **caractérisé en ce que** *le substrat matriciel est carboné et semi-solide, et constitué d'une* matrice cellulosique non-bactéricide imprégnée d'eau déminéralisée et stérile.

2. Procédé d'obtention *in vitro* de biomasses bactériennes édaphiques selon la revendication 1 **caractérisé en ce que** le substrat matriciel carboné semi-solide est constitué d'une matrice cellulosique non-bactéricide imprégnée d'eau déminéralisée et stérile, la quantité d'eau et de matrice étant avantageusement les mêmes afin de constituer une matrice semi-solide contenant initialement 50% de matière sèche (solide) et 50% d'eau (liquide).

3. Procédé d'obtention *in vitro* de biomasses bactériennes édaphiques selon une quelconque des revendications précédentes **caractérisé en ce que** - selon le type de sol et sa teneur en pHBA, un aliquote dilué sera ajouté de façon à assurer un rapport pHBA / oses d'environs 3 à 4, tout en assurant que la teneur en oses soit initialement inférieure à 70 -100 uM.

4. Procédé d'obtention *in vitro* de biomasses bactériennes édaphiques selon une quelconque des revendications précédentes **caractérisé en ce que** le substrat matriciel carboné est a *priori* dépourvu d'oses et d'osides libres assimilables par les (azoto)bactéries du sol.

5. Procédé d'obtention *in vitro* de biomasses bactériennes édaphiques selon la revendication précédente **caractérisé en ce que** le substrat matriciel carboné semi-solide *a priori* dépourvu d'oses et d'osides libres assimilables par les (azoto)bactéries du sol en libère progressivement lors de sa dégradation au cour de cette fermentation à l'état solide, la microflore d'origine tellurique apportée via l'échantillon de sol assurant ainsi la dégradation enzymatique et/ou fongique de la matrice cellulosique.

6. Procédé d'obtention *in vitro* de biomasses bactériennes édaphiques selon une quelconque des revendications précédentes **caractérisé en ce que** l'échantillon de sol est préparé de façon à retenir que les fines particules de sols aux quelles adhèrent les bactéries.

7. Procédé d'obtention *in vitro* de biomasses bactériennes édaphiques selon la revendication précédente **caractérisé en ce que** la granulométrie des fines particules de sols est d'environ 500 um, plus particulièrement d'au plus 270 um, et avantageusement d'environ 70 à 170 um.

8. Procédé d'obtention *in vitro* de biomasses bactériennes édaphiques selon une quelconque des revendications précédentes **caractérisé en ce que** les proportions substrat matriciel carboné semi-solide : échantillon de sol sont comprises entre 25 et 1, plus particulièrement entre 10 et 3, avantageusement d'environ 5.

9. Procédé d'obtention *in vitro* de biomasses bactériennes édaphiques selon une quelconque des revendications précédentes **caractérisé en ce que** le substrat carboné semi-solide ainsi mis en contacte avec lesdits échantillons de sol est incubé suffisamment longtemps pour permettre le développement *in situ* de populations *Azotobacteraceae.*

## Patentansprüche

1. Verfahren zur *in* vitro-Herstellung von bakteriellen Boden-Biomassen, umfassend die folgenden Schritte: (i) In-Kontakt-Bringen einer Bodenprobe mit einem Matrixsubstrat, um einen Bereich auf der Oberfläche des Substrats zu bilden, der analog demjenigen ist, der dem Ton-Humus-Komplex (CAH) zugegeben ist, um auf der Oberfläche oder im Matrixsubstrat das Wesentliche der endogenen bakteriellen Mikroflora der ursprünglichen Bodenmedien zurückzuhalten, (ii) Entwickeln dieses Bereichs unter oligotrophischen Bedingungen, die es den konstitutivsten Zellen des CAH, die dieses Matrixsubstrat kolonisiert haben, ermöglichen, eventuell den Überstand in die flüssige Phase zu migrieren, (iii) Wiedergewinnen und Kultivieren der sich in reichen flüssigen Kulturmedien am meisten vermehrenden Bakterienstämme mit autochthonen, widerstandsfähigen und persistenten Eigenschaften *in situ,* **dadurch gekennzeichnet, dass** das Matrixsubstrat kohlenstoffhaltig und halbfest ist und aus einer nicht bakteriziden Zellulosematrix, imprägniert mit demineralisiertem und sterilem Wasser, besteht.

2. Verfahren zur *in* vitro-Herstellung von bakteriellen Boden-Biomassen nach Anspruch 1, **dadurch gekennzeichnet, dass** das kohlenstoffhaltige und halbfeste Matrixsubstrat aus einer nicht bakteriziden Zellulosematrix, imprägniert mit demineralisiertem und sterilem Wasser, besteht, wobei die Mengen von Wasser und Matrix vorteilhafterweise gleich sind, um eine halbfeste Matrix zu bilden, die anfänglich 50 % Trockenmasse (fest) und 50 % Wasser (flüssig) enthält.

3. Verfahren zur *in* vitro-Herstellung von bakteriellen Boden-Biomassen nach einem der vorhergehenden Ansprüche, **dadurch gekennzeichnet**, dassgemäß der Art des Bodens und seinem Gehalt an pHBA ein verdünntes Aliquot hinzugegeben wird, um eine Verhältnis pHBA/Monosaccharide von ungefähr 3 bis 4 sicherzustellen und gleichzeitig sicherzustellen, dass der Gehalt an Monosacchariden anfänglich weniger als 70 - 100 uM beträgt.

4. Verfahren zur *in* vitro-Herstellung von bakteriellen Boden-Biomassen nach einem der vorhergehenden Ansprüche, **dadurch gekennzeichnet, dass** das kohlenstoffhaltige Matrixsubstrat a priori keine Monosaccharide und freie Saccharide, die von den Azotobakterien des Boden assimiliert werden können, aufweist.

5. Verfahren zur *in* vitro-Herstellung von bakteriellen Boden-Biomassen nach dem vorhergehenden Anspruch, **dadurch gekennzeichnet, dass** das kohlenstoffhaltige und halbfeste Matrixsubstrat, das a priori keine Monosaccharide und freien Saccharide, die von den Azotobakterien des Boden assimiliert werden können, aufweist, daraus fortschreitend bei seinem Abbau im Laufe dieser Fermentierung im festen Zustand die Mikroflora tellurischen Ursprungs, die über die Bodenprobe eingetragen wurde, freigibt, wodurch der enzymatische und/oder mykotische Abbau der Zellulosematrix sichergestellt wird.

6. Verfahren zur *in vitro*-Herstellung von bakteriellen Boden-Biomassen nach einem der vorhergehenden Ansprüche, **dadurch gekennzeichnet, dass** die Bodenprobe derart vorbereitet ist, dass sie nur die feinen Partikel von Böden zurückhält, an die sich die Bakterien anhaften.

7. Verfahren zur *in* vitro-Herstellung von bakteriellen Boden-Biomassen nach einem der vorhergehenden Ansprüche, **dadurch gekennzeichnet, dass** die Granulometrie der feinen Partikel der Böden ungefähr 500 um, insbesondere höchstens 270 um und vorteilhafterweise ungefähr 70 bis 170 um ist.

8. Verfahren zur *in* vitro-Herstellung von bakteriellen Boden-Biomassen nach einem der vorhergehenden Ansprüche, **dadurch gekennzeichnet, dass** die Proportionen zwischen kohlenstoffhaltigem, halbfestem Matrixsubstrat und Bodenprobe zwischen 25 und 1, insbesondere zwischen 10 und 3, vorteilhafterweise ungefähr 5, liegen.

9. Verfahren zur *in vitro*-Herstellung von bakteriellen Boden-Biomassen nach einem der vorhergehenden Ansprüche, **dadurch gekennzeichnet, dass** das halbfeste kohlenstoffhaltige Substrat, das so mit den Bodenproben in Kontakt gebracht wird, ausreichend lange inkubiert wird, um die Entwicklung in situ von Populationen von *Azotobacteraceae* zu ermöglichen.

## Claims

1. Method for obtaining edaphic bacterial biomasses *in vitro,* comprising the steps of: (i) bringing a soil sample into contact with a matrix substrate so as to form on the surface of the substrate a zone analogous to that associated with the argilohumic complex (AHC) in order to retain on the surface or in the matrix substrate the main part of the bacterial microflora endogenous to the source edaphic media, (ii) maturing that zone under oligotrophic conditions allowing the most constitutive cells of the AHC which have colonised the matrix substrate optionally to migrate to the supernatant liquid phase, (iii) recovering and culturing the most prolific bacterial strains in rich liquid culture media, of autochtonous, robust and persistent nature *in situ,* **characterised in that** *the matrix substrate is carbon-containing and semi-solid and composed of a* non-bactericidal cellulose matrix impregnated with demineralised and sterile water.

2. Method for obtaining edaphic bacterial biomasses *in vitro* according to claim 1, **characterised in that** the semi-solid carbon-containing matrix is composed of a non-bactericidal cellulose matrix impregnated with demineralised and sterile water, the quantity of water and matrix advantageously being the same in order to form a semi-solid matrix initially containing 50% dry matter (solid) and 50% water (liquid).

3. Method for obtaining edaphic bacterial biomasses *in vitro* according to either of the preceding claims, **characterised in that** - according to the type of soil and its pHBA content - a diluted aliquot will be added so as to ensure a pHBA/ose ratio of approximately from 3 to 4, while ensuring that the content of oses is initially less than 70 to 100 µM.

4. Method for obtaining edaphic bacterial biomasses *in vitro* according to any one of the preceding claims, **characterised in that** the carbon-containing matrix substrate is *a priori* free of free oses and osides assimilable by the (azoto)bacteria of the soil.

5. Method for obtaining edaphic bacterial biomasses *in vitro* according to the preceding claim, **characterised in that** the semi-solid carbon-containing matrix substrate *a priori* free of free oses and osides assimilable by the (azoto)bacteria of the soil gradually releases them as it is degraded during this fermentation in the solid state, the microflora of telluric origin supplied *via* the soil sample thus ensuring the enzymatic and/or fungal degradation of the cellulose matrix.

6. Method for obtaining edaphic bacterial biomasses *in vitro* according to any one of the preceding claims, **characterised in that** the soil sample is prepared so as to retain only the fine soil particles to which the bacteria adhere.

7. Method for obtaining edaphic bacterial biomasses *in vitro* according to the preceding claim, **characterised in that** the particle size of the fine soil particles is approximately 500 µm, more particularly not more than 270 µm and advantageously approximately from 70 to 170 µm.

8. Method for obtaining edaphic bacterial biomasses *in vitro* according to any one of the preceding claims, **characterised in that** the proportions semi-solid carbon-containing matrix substrate : soil sample are between 25 and 1, more particularly between 10 and 3, advantageously approximately 5.

9. Method for obtaining edaphic bacterial biomasses *in vitro* according to any one of the preceding claims, **characterised in that** the semi-solid carbon-containing substrate thus brought into contact with said soil samples is incubated for a sufficient length of time to permit the *in situ* development of *Azotobacteraceae* populations.
